# EUROPEAN PATENT APPLICATION

(11) **EP 4 193 985 A1**
(43) Date of publication of application: **14.06.2023**
(21) Application number: 21853295.0
(22) Date of filing: 06.08.2021
(51) Int. Cl.: A61K 6/40, A61C 7/00

(54) **COMPOSITION FOR DENTAL ATTACHMENT**

(30) Priority: 07.08.2020 JP 2020135259
(71) Applicant: Kuraray Noritake Dental Inc., Kurashiki-shi, Okayama 710-0801 (JP)
(72) Inventor: MATSUURA, Ryo, Tainai-shi, Niigata 959-2653 (JP); NOJIRI, Yamato, Tainai-shi, Niigata 959-2653 (JP)
(74) Representative: D Young & Co LLP
(86) International application number: PCT/JP2021/029456
(87) International publication number: WO 2022/030642

(57) **Abstract**

The present invention provides a composition for dental attachments that exhibits excellent adhesive properties to uncut enamel without carrying out a pretreatment with, for example, a dental adhesive after etching with phosphoric acid or the like, and that has more than a certain level of mechanical characteristics, and enables a simplified bonding procedure for dental attachments. The present invention relates to a composition for dental attachments that comprises a polymerizable monomer (A), a photopolymerization initiator (B), and a filler (C), the polymerizable monomer (A) comprising a polymerizable monomer (A-1) having an acidic group, and a polymerizable monomer (A-2) having no acidic group, the content of the polymerizable monomer (A-1) having an acidic group being 1 to 40 parts by mass in total 100 parts by mass of the polymerizable monomer (A), the content of the filler (C) being 50 to 90 parts by mass in total 100 parts by mass of the composition.

## Description

### TECHNICAL FIELD

The present invention relates to a composition for dental attachments. More specifically, the present invention relates to a composition for dental attachments that exhibits excellent adhesive properties to uncut enamel without carrying out a pretreatment with, for example, a dental adhesive after etching with phosphoric acid or the like, and that has more than a certain level of mechanical characteristics.

### BACKGROUND ART

Traditionally, brackets are in the orthodontic mainstream. However, because of the unnatural appearance of brackets, there is growing popularity of an orthodontic treatment that uses a colorless transparent mouthpiece, or an orthodontic aligner as it is commonly called (hereinafter, also referred to simply as "aligner"). In orthodontic treatment with brackets, brackets are attached to tooth surfaces with their shape to hold wires. The wires held by the brackets apply a mechanical load (hereinafter, also referred to as "orthodontic forces") to guide the teeth to desired positions. An orthodontic adhesive is commonly used to attach brackets to tooth surfaces. In aligner orthodontics, an aligner is worn that is similar in shape to a mouthpiece. In a known method of aligner orthodontics, "bumps", or attachments, are similarly formed on tooth surfaces, and an aligner, by being hooked to the bumps, applies a mechanical load that is more adequate to more efficiently guide teeth to desired positions. Such orthodontic adhesives and attachments typically use dental materials made of a curable composition containing components such as a polymerizable monomer (e.g., a (meth)acrylic acid ester), a polymerization initiator, and a filler. Specifically, dental composite resins are commonly used as material of attachments.

Attachments are formed by a method that uses a mouthpiece called a template reflecting the position of the dentition before orthodontic treatment. The following describes a typical method of forming attachments using a template. A template has depressions having the shapes of attachments in locations corresponding to tooth surfaces. First, these depressions are filled with a dental composite resin. As a pretreatment, tooth surfaces selectively receive a surface treatment (etching) with phosphoric acid or the like in places where attachments need to be formed. An orthodontic adhesive is then applied to these portions, and is cured by photoirradiation or other methods after optionally removing solvent by blowing air. The template is attached to teeth with the dental composite resin filling the depressions, and the dental composite resin filling the depressions in the shapes of attachments is cured by photoirradiation or other methods to form attachments at the desired locations on tooth surfaces (the locations where the dental adhesive was applied). That is, a technique is widely known that forms aligner attachments by bonding a dental composite resin to uncut enamel with a dental adhesive after phosphoric acid etching (see, for example, Non Patent Literatures 1 and 2).

In orthodontic treatment using aligners, the attachments must withstand various types of loads, including the load applied while wearing the aligner, the load applied when inserting or removing the aligner, the load applied by brushing, and the load of tooth flexure due to biting, so that the attachments do not come off the surfaces of tooth structure. However, there is a possibility of attachments coming off the surfaces during treatment when the material has low adhesive properties to uncut enamel after phosphoric acid etching. Another issue is that the material, when it has low strength and low elastic modulus in its cured product, may cause damage to the attachments during orthodontic treatment, or may fail to provide good fit to the aligner to enable a proper orthodontic treatment. Certain levels of strength and elastic modulus are needed to avoid such a problem.

An orthodontic treatment with an aligner requires bonding numerous attachments to the tooth structure. The methods that use dental adhesives disclosed in Non Patent Literatures 1 and 2 involve complex procedures because the methods require phosphoric acid etching and application of a bonding agent for every tooth surface in need of attachment formation, in addition to filling a template with a curable dental material. Ideally, a dental adhesive is selectively applied only to portions where the attachments must bond the tooth structure. However, aside from the fact that selective application of a dental adhesive to the specified locations itself is difficult, it is practically impossible to avoid scattering of dental adhesive to areas other than the attachment areas of tooth surfaces in evaporating the solvent of dental adhesive by air blowing. The areas of tooth structure where the dental adhesive has adhered is susceptible to staining and adhesion of bacteria, and a much longer treatment time is needed if the dental adhesive were to be removed from areas other than the areas of tooth surface where the attachments are to be attached. As discussed above, formation of attachments involves a major drawback when the procedure uses a dental adhesive. This is greatly different from a common restorative filling treatment of tooth cavities.

It may be conceivable to bring orthodontic adhesives to use as attachment material, using, for example, the orthodontic adhesives used for orthodontic purposes disclosed in Patent Literatures 1 to 4.

### CITATION LIST

### Patent Literature

Patent Literature 1: WO2015/141683
Patent Literature 2: JP 2010-46266 A
Patent Literature 3: JP 2011-207806 A
Patent Literature 4: JP 2016-6040 A

### Non Patent Literature

Non Patent Literature 1: ACTA ODONTOLOGICA LATINOAMERICANA, 2017, Vol. 30, Issue 2, pp 90-95
Non Patent Literature 2: Materials, Changes in Roughness and Mechanical Properties of Invisalign Appliances after One- and Two-Weeks Use, 2019, Vol. 12(15), 2406

### SUMMARY OF INVENTION

### Technical Problem

An investigation by the present inventors revealed that the orthodontic adhesives described in Patent Literatures 1 and 2 do not contain a polymerizable monomer having an acidic group, and require further improvement of adhesive properties to uncut enamel after phosphoric acid etching. The dental curable compositions described in Patent Literatures 3 and 4 are suited as fixing materials for loose teeth because cured products of these compositions have a small elastic modulus. However, the dental curable compositions described in Patent Literatures 3 and 4 are clearly not attachment materials that enable a proper orthodontic treatment because the orthodontic force applied to teeth from an aligner greatly decreases as it acts through attachments made of these dental curable compositions.

It is an object of the present invention to provide a composition for dental attachments that exhibits excellent adhesive properties to uncut enamel without carrying out a pretreatment with, for example, a dental adhesive after etching with phosphoric acid or the like, and that has more than a certain level of mechanical characteristics, and enables a simplified bonding procedure for dental attachments.

### Solution to Problem

Specifically, the present invention includes the following.
[1] A composition for dental attachments, comprising a polymerizable monomer (A), a photopolymerization initiator (B), and a filler (C),
   the polymerizable monomer (A) comprising a polymerizable monomer (A-1) having an acidic group, and a polymerizable monomer (A-2) having no acidic group,
   the content of the polymerizable monomer (A-1) having an acidic group being 1 to 40 parts by mass in total 100 parts by mass of the polymerizable monomer (A),
   the content of the filler (C) being 50 to 90 parts by mass in total 100 parts by mass of the composition.
[2] The composition for dental attachments according to [1], wherein the polymerizable monomer (A-2) having no acidic group comprises a hydrophobic polymerizable monomer (A-2b) having no acidic group, and, optionally, a hydrophilic polymerizable monomer (A-2c) having no acidic group,
   the hydrophilic polymerizable monomer (A-2c) having no acidic group and the hydrophobic polymerizable monomer (A-2b) having no acidic group having a mass ratio of 0:10 to 2:1 as a mass ratio of the hydrophilic polymerizable monomer (A-2c) having no acidic group to the hydrophobic polymerizable monomer (A-2b) having no acidic group.
[3] The composition for dental attachments according to [1] or [2], wherein the composition for dental attachments is of a one-pack type.
[4] The composition for dental attachments according to any one of [1] to [3], wherein the polymerizable monomer (A-1) having an acidic group is a polymerizable monomer having a phosphoric acid group, and/or a polymerizable monomer having a carboxylic acid group.
[5] The composition for dental attachments according to any one of [1] to [4], wherein the polymerizable monomer (A-1) having an acidic group is 10-methacryloyloxydecyl dihydrogenphosphate.
[6] The composition for dental attachments according to any one of [2] to [5], wherein the hydrophilic polymerizable monomer (A-2c) having no acidic group and the hydrophobic polymerizable monomer (A-2b) having no acidic group have a mass ratio of 0:10 to 1:1 as a mass ratio of the hydrophilic polymerizable monomer (A-2c) having no acidic group to the hydrophobic polymerizable monomer (A-2b) having no acidic group.
[7] The composition for dental attachments according to any one of [2] to [5], wherein the hydrophilic polymerizable monomer (A-2c) having no acidic group and the hydrophobic polymerizable monomer (A-2b) having no acidic group have a mass ratio of 0:10 to 1:2 as a mass ratio of the hydrophilic polymerizable monomer (A-2c) having no acidic group to the hydrophobic polymerizable monomer (A-2b) having no acidic group.
[8] The composition for dental attachments according to any one of [1] to [7], wherein the filler (C) comprises at least one combination selected from the group consisting of:
   a combination (I) of a filler (C-1) having an average particle diameter of 1 nm or more and less than 0.1 µm, and a filler (C-2) having an average particle diameter of 0.1 µm or more and 1 µm or less;
   a combination (II) of a filler (C-1) having an average particle diameter of 1 nm or more and less than 0.1 µm, and a filler (C-3) having an average particle diameter of more than 1 µm and 10 µm or less;
   a combination (III) of a filler (C-1) having an average particle diameter of 1 nm or more and less than 0.1 µm, a filler (C-2) having an average particle diameter of 0.1 µm or more and 1 µm or less, and a filler (C-3) having an average particle diameter of more than 1 µm and 10 µm or less; and
   a combination (IV) of fillers (C-2) having an average particle diameter of 0.1 µm or more and 1 µm or less.
[9] The composition for dental attachments according to [8], wherein the filler (C) comprises the combination (I) or the combination (II).
[10] The composition for dental attachments according to any one of [1] to [9], wherein a cured product of the composition has a flexural modulus of 3 GPa or more.
[11] The composition for dental attachments according to any one of [1] to [10], wherein the photopolymerization initiator (B) comprises a water-soluble photopolymerization initiator (B-1).
[12] The composition for dental attachments according to any one of [1] to [11], wherein the photopolymerization initiator (B) comprises a water-insoluble photopolymerization initiator (B-2).
[13] The composition for dental attachments according to any one of [1] to [12], wherein the polymerizable monomer (A-2) having no acidic group comprises an asymmetric acrylamide-methacrylic acid ester compound (A-2a) represented by the following general formula (1), 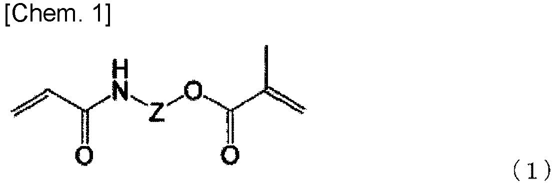 wherein Z is an optionally substituted C₁ to Ca linear or branched aliphatic group, or an optionally substituted aromatic group, and the aliphatic group may be interrupted by at least one binding group selected from the group consisting of -O-, -S-, -CO-, -CO-O-, -O-CO-, -NR¹-, -CO-NR'-, -NR'-CO-, -CO-O-NR'-, -O-CO-NR¹-, and -NR'-CO-NR'-, where R¹ represents a hydrogen atom, or an optionally substituted C₁ to Ca linear or branched aliphatic group.
[14] The composition for dental attachments according to [13], wherein Z is an optionally substituted C₁ to C₄ linear or branched aliphatic group.
[15] The composition for dental attachments according to [13] or [14], wherein Z is an optionally substituted C₁ to C₄ linear or branched alkylene group.
[16] The composition for dental attachments according to any one of [13] to [15], wherein the asymmetric acrylamide-methacrylic acid ester compound (A-2a) represented by the general formula (1) is at least one selected from the group consisting of N-methacryloyloxyethylacrylamide, N-methacryloyloxypropylacrylamide, N-methacryloyloxybutylacrylamide, N-(1-ethyl-(2-methacryloyloxy)ethyl)acrylamide, and N-(2-(2-methacryloyloxyethoxy)ethyl)acrylamide.

### Advantageous Effects of Invention

According to the present invention, a composition for dental attachments can be provided that exhibits excellent adhesive properties to uncut enamel without carrying out a pretreatment with, for example, a dental adhesive after etching with phosphoric acid or the like, and that has more than a certain level of mechanical characteristics, and enables a simplified bonding procedure for dental attachments. A composition for dental attachments of the present invention can be suitably used as a dental attachment for aligner orthodontics. A composition for dental attachments of the present invention exhibits excellent adhesive properties without using a dental adhesive, and can simplify the orthodontic treatment procedures. Because there is no need to use a dental adhesive, another advantageous effect of the present invention is that it does not require the procedure to remove a dental adhesive that has scattered to areas other than the target tooth surfaces.

### BRIEF DESCRIPTION OF DRAWINGS

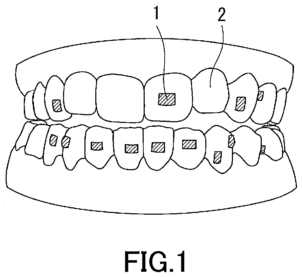

FIG. 1 is a schematic diagram of dental attachments according to an embodiment of the present invention.

### DESCRIPTION OF EMBODIMENTS

A composition for dental attachments of the present invention comprises a polymerizable monomer (A), a photopolymerization initiator (B), and a filler (C), the polymerizable monomer (A) comprising a polymerizable monomer (A-1) having an acidic group, and a polymerizable monomer (A-2) having no acidic group, the content of the polymerizable monomer (A-1) having an acidic group being 1 to 40 parts by mass in total 100 parts by mass of the polymerizable monomer (A), the content of the filler (C) being 50 to 90 parts by mass in total 100 parts by mass of the composition for dental attachments. In this specification, "dental attachment" means an orthodontic attachment, that is, an orthodontic component used with an orthodontic aligner. FIG. 1 shows a schematic diagram of dental attachments. For example, as shown in FIG. 1, the dental attachments 1 are formed as bumps on surfaces of teeth 2, which include uncut natural teeth, and an orthodontic aligner is hooked to the bumps to apply a mechanical load that is more adequate to more efficiently guide teeth to desired positions.

In view of adhesive properties to tooth structure, a composition for dental attachments of the present invention has a depth of photocure of preferably 2 mm or more, more preferably 2.5 mm or more. The upper limit of depth of photocure is not particularly limited, and may be, for example, 6 mm or less. At least a certain thickness is required for dental attachments to properly apply orthodontic forces. With at least a certain depth of photocure, high adhesive properties can be obtained because, even when attachments have more than a certain thickness, the composition for dental attachments can sufficiently cure at its bonding interface with the tooth structure when cured. As a rule, a photocurable material tends to have a low mechanical strength because curing does not sufficiently proceed in deeper portions where the intensity of light is weaker. In the case of a restorative filling treatment, the farthest point on the depth of photocure corresponds to the bottom of a cavity, and is unlikely to be affected by a thermal load or wearing due to toothbrushing. However, in dental attachments, such an area is exposed to the surface of tooth structure, and the influence of thermal load or wearing due to toothbrushing is considerably large in portions where the mechanical strength is weaker. Accordingly, a high depth of photocure is required to provide high strength also in deeper portions of the material. That is, a circumstance exists that requires dental attachments to have a high depth of photocure, despite being formed on tooth surfaces. The method of measurement of a depth of photocure is as described in the EXAMPLES section below.

In view of the strength of dental attachments, it is preferable that a cured product of a composition for dental attachments of the present invention have a Vickers hardness of 30 Hv or more, more preferably 33 Hv or more, even more preferably 35 Hv or more after being cured by applying light for 10 seconds with a dental LED irradiator. By having a high Vickers hardness, the dental attachments can be more resistant to wear against, for example, the frictional force exerted when inserting or removing an aligner or when brushing teeth, and the cured product can exhibit excellent strength to maintain orthodontic forces also as dental attachments. Unlike filling composite resins, removal of dental attachments is difficult when the Vickers hardness is too high. It is therefore preferable, in view of ease of removal of dental attachments, that the upper limit of Vickers hardness be 70 Hv or less, more preferably 65 Hv or less, even more preferably 60 Hv or less. The method of measurement of the Vickers hardness of a cured product is as described in the EXAMPLES section below.

In view of the strength of dental attachments, it is preferable that a cured product of a composition for dental attachments of the present invention have a flexural modulus of 3 GPa or more, more preferably 3.5 GPa or more. Even more preferably, the flexural modulus is 4.0 GPa or more because it enables a cured product of a composition for dental attachments of the present invention to exhibit even stronger orthodontic forces as an orthodontic appliance, jointly with the orthodontic aligner used together. In certain preferred embodiments, a cured product of a composition for dental attachments of the present invention may have a flexural modulus of 5.0 GPa or more, or 5.5 GPa or more. By having more than a certain level of flexural modulus, the dental attachments can be more resistant to deformation that occurs when, for example, inserting or removing an aligner, or brushing teeth. In view of ease of removal of dental attachments, the flexural modulus is preferably less than 10.0 GPa, more preferably less than 9.5 GPa, even more preferably less than 9.0 GPa. The method of measurement of the flexural modulus of a cured product is as described in the EXAMPLES section below.

In view of the strength of dental attachments, it is preferable that a cured product of a composition for dental attachments of the present invention have a three-point flexural strength of 70 MPa or more, more preferably 75 MPa or more. Even more preferably, the three-point flexural strength is 80 MPa or more because it enables a cured product of a composition for dental attachments of the present invention to exhibit even stronger orthodontic forces as an orthodontic appliance, jointly with the orthodontic aligner used together. In certain preferred embodiments, a cured product of a composition for dental attachments of the present invention may have a three-point flexural strength of 85 MPa or more, or 88 MPa or more. By having more than a certain level of flexural strength, the dental attachments can be more resistant to deformation that occurs when, for example, inserting or removing an aligner, or brushing teeth. In view of ease of removal of dental attachments, the flexural strength is preferably less than 200 MPa, more preferably less than 180 MPa, even more preferably less than 160 MPa. The method of measurement of the three-point flexural strength of a cured product is as described in the EXAMPLES section below.

In dental attachments, higher adhesive properties and bond durability are required than in common filling applications because the bonding interface is always exposed at the surface of tooth structure, and, in addition to dental attachments requiring a thickness of about 2 mm, the outermost surface of enamel is more acid resistant than the enamel in the tooth structure, and cannot be demineralized as easily by etching, and the surface of uncut enamel tends to have a lower bond strength than cut enamel surfaces, when compared under the condition that the cured product after polymerization has more than a certain thickness (for example, a thickness of 2 mm or more). Concerning the initial bond strength, it is therefore preferable that a composition for dental attachments of the present invention, when its cured product has a thickness of 2 mm, have a shear bond strength of 15 MPa or more, more preferably 16 MPa or more, even more preferably 18 MPa or more for uncut enamel after phosphoric acid etching. The method of measurement of shear bond strength concerning initial bond strength is as described in the EXAMPLES section below. Concerning bond durability, it is preferable that a composition for dental attachments of the present invention, when its cured product has a thickness of 2 mm, have a shear bond strength of 15 MPa or more, more preferably 18 MPa or more, even more preferably 20 MPa or more for uncut enamel after phosphoric acid etching when subjected to 10,000 thermal cycles under the conditions given in the EXAMPLES section below. The method of measurement of shear bond strength concerning bond durability is as described in the EXAMPLES section below. A composition for dental attachments of the present invention has high adhesive properties and high bond durability also for zirconia (zirconia sintered body) or a gold silver palladium alloy. Concerning initial bond strength, a composition for dental attachments of the present invention has a tensile bond strength of preferably 15 MPa or more, more preferably 16 MPa or more, even more preferably 18 MPa or more for zirconia. Concerning bond durability, a composition for dental attachments of the present invention has a tensile bond strength of preferably 10 MPa or more, more preferably 12 MPa or more, even more preferably 14 MPa or more for zirconia. Concerning initial bond strength, a composition for dental attachments of the present invention has a tensile bond strength of preferably 10 MPa or more, more preferably 12 MPa or more, even more preferably 13 MPa or more for a gold silver palladium alloy. Concerning bond durability, a composition for dental attachments of the present invention has a tensile bond strength of preferably 8 MPa or more, more preferably 9 MPa or more, even more preferably 10 MPa or more for a gold silver palladium alloy.

In dental attachments, the effect of water absorption is greater than in common filling applications because the cured product is always exposed at the surface of tooth structure. As a rule, more water is absorbed when a composition contains a polymerizable monomer having an acidic group, and the amount of water absorption needs to be reduced in such a composition while containing a polymerizable monomer having an acidic group. Concerning water absorption, it is therefore preferable that a composition for dental attachments of the present invention have a water absorption of 40 µg/mm³ or less, more preferably 30 µg/mm³ or less, even more preferably 20 µg/mm³ or less, as measured by a testing method in compliance with ISO 4049:2009.

### Polymerizable Monomer (A)

Preferably, a radical polymerizable monomer is used as the polymerizable monomer (A) used in a composition for dental attachments of the present invention. Specific examples of the radical polymerizable monomer as polymerizable monomer (A) include (meth)acrylate polymerizable monomers; (meth)acrylamide polymerizable monomers; esters, vinyl esters, and vinyl ethers of α-cyanoacrylic acid, (meth)acrylic acid, α-halogenated acrylic acid, crotonic acid, cinnamic acid, sorbic acid, maleic acid, and itaconic acid; mono-N-vinyl derivatives; and styrene derivatives. In view of curability, preferred are (meth)acrylate polymerizable monomers and (meth)acrylamide polymerizable monomers. In view of adhesive properties to tooth structure and elastic modulus, the polymerizable monomer (A) in a composition for dental attachments of the present invention must comprise a polymerizable monomer (A-1) having an acidic group, and a polymerizable monomer (A-2) having no acidic group.

### Polymerizable Monomer (A-1) Having Acidic Group

The polymerizable monomer (A-1) having an acidic group used in the present invention may be, for example, a polymerizable monomer having at least one acidic group such as a phosphoric acid group, a pyrophosphoric acid group, a thiophosphoric acid group, a phosphonic acid group, a carboxylic acid group, or a sulfonic acid group. The polymerizable monomer (A-1) having an acidic group may be used alone, or two or more thereof may be used in appropriate combinations. Specific examples of the polymerizable monomer (A-1) having an acidic group are as follows.

Examples of the polymerizable monomer having a phosphoric acid group include 2-(meth)acryloyloxyethyl dihydrogenphosphate, 3-(meth)acryloyloxypropyl dihydrogenphosphate, 4-(meth)acryloyloxybutyl dihydrogenphosphate, 5-(meth)acryloyloxypentyl dihydrogenphosphate, 6-(meth)acryloyloxyhexyl dihydrogenphosphate, 7-(meth)acryloyloxyheptyl dihydrogenphosphate, 8-(meth)acryloyloxyoctyl dihydrogenphosphate, 9-(meth)acryloyloxynonyl dihydrogenphosphate, 10-(meth)acryloyloxydecyl dihydrogenphosphate, 11-(meth)acryloyloxyundecyl dihydrogenphosphate, 12-(meth)acryloyloxydodecyl dihydrogenphosphate, 16-(meth)acryloyloxyhexadecyl dihydrogenphosphate, 20-(meth)acryloyloxyicosyl dihydrogenphosphate, bis[2-(meth)acryloyloxyethyl]hydrogenphosphate, bis[4-(meth)acryloyloxybutyl]hydrogenphosphate, bis[6-(meth)acryloyloxyhexyl]hydrogenphosphate, bis[8-(meth)acryloyloxyoctyl]hydrogenphosphate, bis[9-(meth)acryloyloxynonyl]hydrogenphosphate, bis[10-(meth)acryloyloxydecyl]hydrogenphosphate, 1,3-di(meth)acryloyloxypropyl dihydrogenphosphate, 2-(meth)acryloyloxyethylphenyl hydrogenphosphate, 2-(meth)acryloyloxyethyl-(2-bromoethyl)hydrogenphosphate, 2-methacryloyloxyethyl-(4-methoxyphenyl)hydrogenphosphate, 2-methacryloyloxypropyl-(4-methoxyphenyl)hydrogenphosphate, and acid chlorides, alkali metal salts, and amine salts of these.

Examples of the polymerizable monomer having a pyrophosphoric acid group include bis[2-(meth)acryloyloxyethyl]pyrophosphate, bis[4-(meth)acryloyloxybutyl]pyrophosphate, bis[6-(meth)acryloyloxyhexyl]pyrophosphate, bis[8-(meth)acryloyloxyoctyl]pyrophosphate, bis[10-(meth)acryloyloxydecyl]pyrophosphate, and acid chlorides, alkali metal salts, and amine salts of these.

Examples of the polymerizable monomer having a thiophosphoric acid group include 2-(meth)acryloyloxyethyl dihydrogenthiophosphate, 3-(meth)acryloyloxypropyl dihydrogenthiophosphate, 4-(meth)acryloyloxybutyl dihydrogenthiophosphate, 5-(meth)acryloyloxypentyl dihydrogenthiophosphate, 6-(meth)acryloyloxyhexyl dihydrogenthiophosphate, 7-(meth)acryloyloxyheptyl dihydrogenthiophosphate, 8-(meth)acryloyloxyoctyl dihydrogenthiophosphate, 9-(meth)acryloyloxynonyl dihydrogenthiophosphate, 10-(meth)acryloyloxydecyl dihydrogenthiophosphate, 11-(meth)acryloyloxyundecyl dihydrogenthiophosphate, 12-(meth)acryloyloxydodecyl dihydrogenthiophosphate, 16-(meth)acryloyloxyhexadecyl dihydrogenthiophosphate, 20-(meth)acryloyloxyicosyl dihydrogenthiophosphate, and acid chlorides, alkali metal salts, and ammonium salts of these.

Examples of the polymerizable monomer having a phosphonic acid group include 2-(meth)acryloyloxyethylphenyl phosphonate, 5-(meth)acryloyloxypentyl-3-phosphonopropionate, 6-(meth)acryloyloxyhexyl-3-phosphonopropionate, 10-(meth)acryloyloxydecyl-3-phosphonopropionate, 6-(meth)acryloyloxyhexyl-3-phosphonoacetate, 10-(meth)acryloyloxydecyl-3-phosphonoacetate, and acid chlorides, alkali metal salts, and ammonium salts of these.

Examples of the polymerizable monomer having a carboxylic acid group include a monofunctional (meth)acrylic acid ester having one carboxyl group or acid anhydride group thereof within the molecule, and a monofunctional (meth)acrylic acid ester having a plurality of carboxyl groups or acid anhydride groups thereof within the molecule.

Examples of the monofunctional polymerizable monomer having one carboxyl group or acid anhydride group thereof within the molecule include (meth)acrylic acid, N-(meth)acryloylglycine, N-(meth)acryloylaspartic acid, 2-(meth)acryloyloxyethyl hydrogen succinate, 2-(meth)acryloyloxyethyl hydrogen phthalate, 2-(meth)acryloyloxyethyl hydrogen malate, O-(meth)acryloyltyrosine, N-(meth)acryloyltyrosine, N-(meth)acryloylphenylalanine, N-(meth)acryloyl-p-aminobenzoic acid, N-(meth)acryloyl-o-aminobenzoic acid, 2-(meth)acryloyloxybenzoic acid, 3-(meth)acryloyloxybenzoic acid, 4-(meth)acryloyloxybenzoic acid, N-(meth)acryloyl-5-aminosalicylic acid, N-(meth)acryloyl-4-aminosalicylic acid, and compounds in which the carboxyl groups of the foregoing compounds have been converted into acid anhydride groups.

Examples of the monofunctional polymerizable monomer having a plurality of carboxyl groups or acid anhydride groups thereof within the molecule include 6-(meth)acryloyloxyhexane-1 , 1 -dicarboxylic acid, 9-(meth)acryloyloxynonane-1, 1-dicarboxylic acid, 10-(meth)acryloyloxydecane-1,1 -dicarboxylic acid, 11- (meth)acryloyloxyundecane-1,1-dicarboxylic acid, 12-(meth)acryloyloxydodecane-1,1-dicarboxylic acid, 13-(meth)acryloyloxytridecane-1,1-dicarboxylic acid, 4-(meth)acryloyloxyethyl trimellitate, 4-(meth)acryloyloxyethyl trimellitate anhydride, 4-(meth)acryloyloxybutyl trimellitate, 4-(meth)acryloyloxyhexyl trimellitate, 4-(meth)acryloyloxydecyl trimellitate, 2-(meth)acryloyloxyethyl-3'-(meth)acryloyloxy-2'-(3,4-dicarboxybenzoyloxy)propylsuccinate, 6-(meth)acryloyloxyethylnaphthalene-1,2,6-tricarboxylic acid anhydride, 6-(meth)acryloyloxyethylnaphthalene-2,3,6-tricarboxylic acid anhydride, 4-(meth)acryloyloxyethylcarbonylpropionoyl-1,8-naphthalic acid anhydride, and 4-(meth)acryloyloxyethylnaphthalene-1,8-tricarboxylic acid anhydride.

Examples of the polymerizable monomer having a sulfonic acid group include 2-sulfoethyl (meth)acrylate.

In view of the desirable bond strength of when the composition is used as a composition for dental attachments, the polymerizable monomer (A-1) having an acidic group preferably comprises a polymerizable monomer having a phosphoric acid group, or a polymerizable monomer having a carboxylic acid group, and more preferably comprises 2-(meth)acryloyloxyethyl dihydrogenphosphate, 3-(meth)acryloyloxypropyl dihydrogenphosphate, 4-(meth)acryloyloxybutyl dihydrogenphosphate, 5-(meth)acryloyloxypentyl dihydrogenphosphate, 6-(meth)acryloyloxyhexyl dihydrogenphosphate, 7-(meth)acryloyloxyheptyl dihydrogenphosphate, 8-(meth)acryloyloxyoctyl dihydrogenphosphate, 9-(meth)acryloyloxynonyl dihydrogenphosphate, 10-(meth)acryloyloxydecyl dihydrogenphosphate, 11-(meth)acryloyloxyundecyl dihydrogenphosphate, 12-(meth)acryloyloxydodecyl dihydrogenphosphate, 16-(meth)acryloyloxyhexadecyl dihydrogenphosphate, 20-(meth)acryloyloxyicosyl dihydrogenphosphate, 4-(meth)acryloyloxyethyl trimellitate anhydride, 4-(meth)acryloyloxyethyl trimellitate, 11-(meth)acryloyloxyundecane-1,1-dicarboxylic acid, or a mixture of 2-methacryloyloxyethyl dihydrogenphosphate and bis(2-methacryloyloxyethyl)hydrogenphosphate. In dental attachments, the bonding interface is always exposed at the surfaces of tooth structure, and the polymerizable monomer (A-1) having an acidic group requires higher water resistance than in common restorative filling applications. More preferred from this perspective are 8-(meth)acryloyloxyoctyl dihydrogenphosphate, 9-(meth)acryloyloxynonyl dihydrogenphosphate, 10-(meth)acryloyloxydecyl dihydrogenphosphate, 11-(meth)acryloyloxyundecyl dihydrogenphosphate, 12-(meth)acryloyloxydodecyl dihydrogenphosphate, 16-(meth)acryloyloxyhexadecyl dihydrogenphosphate, and 20-(meth)acryloyloxyicosyl dihydrogenphosphate. In view of a balance with curability, 10-(meth)acryloyloxydecyl dihydrogenphosphate is most preferred.

In view of adhesive properties to uncut enamel after etching, the content of the polymerizable monomer (A-1) having an acidic group in a composition for dental attachments of the present invention needs to be 1 to 40 parts by mass in total 100 parts by mass of polymerizable monomer (A). Preferably, the content is 2.5 to 35 parts by mass, more preferably 5 to 30 parts by mass.

### Polymerizable Monomer (A-2) Having No Acidic Group

Examples of the polymerizable monomer (A-2) having no acidic group in the present invention include an asymmetric acrylamide-methacrylic acid ester compound (A-2a); a hydrophobic polymerizable monomer (A-2b) having no acidic group and having a solubility of less than 10 mass% in 25°C water; and a hydrophilic polymerizable monomer (A-2c) having no acidic group and having a solubility of 10 mass% or more in 25°C water. The polymerizable monomer (A-2) having no acidic group may be used alone, or two or more thereof may be used in combination. In the present invention, the asymmetric acrylamide-methacrylic acid ester compound (A-2a) represents a compound having no acidic group and containing an acrylamide group and a methacryloyloxy group, and compounds containing no acidic group and that do not classify as asymmetric acrylamide-methacrylic acid ester compound (A-2a) are divided into hydrophobic polymerizable monomer (A-2b) and hydrophilic polymerizable monomer (A-2c), depending on the degree of hydrophilicity.

### Asymmetric Acrylamide-Methacrylic Acid Ester Compound (A-2a)

A certain preferred embodiment is, for example, a composition for dental attachments that additionally comprises an asymmetric acrylamide-methacrylic acid ester compound (A-2a). Because of the ability to improve the adhesive properties to tooth structure or other properties of the composition for dental attachments, the asymmetric acrylamide-methacrylic acid ester compound (A-2a) is preferably a compound represented by the following general formula (1).

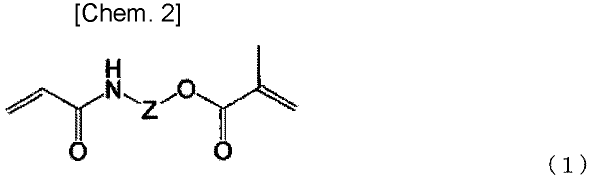

In the formula, Z is an optionally substituted C₁ to C₈ linear or branched aliphatic group, or an optionally substituted aromatic group, and the aliphatic group may be interrupted by at least one binding group selected from the group consisting of -O-, -S-, - CO-, -CO-O-, -O-CO-, -NR¹-, -CO-NR'-, -NR'-CO-, -CO-O-NR¹-, -O-CO-NR¹-, and -NR¹-CO-NR¹-. R¹ represents a hydrogen atom, or an optionally substituted C₁ to Ca linear or branched aliphatic group.

Z is a moiety that adjusts the hydrophilicity of the asymmetric acrylamide-methacrylic acid ester compound (A-2a). The optionally substituted C₁ to Ca aliphatic group represented by Z may be a saturated aliphatic group (an alkylene group, or a cycloalkylene group (for example, a 1,4-cyclohexylene group)), or an unsaturated aliphatic group (an alkenylene group or an alkynylene group). In view of availability or ease of production, and chemical stability, the optionally substituted C₁ to Ca aliphatic group is preferably a saturated aliphatic group (an alkylene group). In view of adhesive properties to tooth structure and polymerization curability, Z is preferably an optionally substituted linear or branched C₁ to C₄ aliphatic group, more preferably an optionally substituted linear or branched C₂ to C₄ aliphatic group. The aliphatic group is preferably an alkylene group. Examples of the C₁ to Ca alkylene group include a methylene group, an ethylene group, a n-propylene group, an isopropylene group, and a n-butylene group.

Examples of the optionally substituted aromatic group represented by Z include an aryl group, and an aromatic heterocyclic group. The aromatic group is preferably an aryl group. The heteroring of the aromatic heterocyclic group is normally unsaturated. The aromatic heteroring is preferably a five-membered ring or a six-membered ring. Preferably, the aryl group is, for example, a phenyl group. Examples of the aromatic heterocyclic group include a furan group, a thiophene group, a pyrrole group, an oxazole group, an isoxazole group, a thiazole group, an isothiazole group, an imidazole group, a pyrazole group, a furazan group, a triazole group, a pyran group, a pyridine group, a pyridazine group, a pyrimidine group, a pyrazine group, and a 1,3,5-triazine group. The aromatic group is particularly preferably a phenyl group.

The aliphatic group represented by R¹ may be a saturated aliphatic group (an alkyl group) or an unsaturated aliphatic group (an alkenyl group or an alkynyl group). In view of availability or ease of production, and chemical stability, the aliphatic group represented by R¹ is preferably a saturated aliphatic group (an alkyl group). The alkyl group may be, for example, any of the alkyl groups presented as substituents of X.

More preferred as R¹ is a hydrogen atom, or an optionally substituted linear or branched C₁ to C₄ alkyl group, even more preferably a hydrogen atom, or an optionally substituted linear or branched C₁ to C₃ alkyl group.

When the aliphatic group represented by Z is interrupted by the above binding group, the number of binding groups is not particularly limited, and may be about 1 to 10, preferably 1, 2, or 3, more preferably 1 or 2. In the formula (1), the aliphatic group represented by Z is preferably one that is not contiguously interrupted by the binding group. That is, the aliphatic group represented by Z is preferably one in which the binding groups are not adjacent to each other. The binding group is more preferably at least one selected from the group consisting of -O-, -S-, -CO-, -CO-O-, -O-CO-, -NH-, - CO-NH-, -NH-CO-, -CO-O-NH-, -O-CO-NH-, and -NH-CO-NH-, particularly preferably at least one selected from the group consisting of -O-, -S-, -CO-, -NH-, -CO-NH-, and -NH-CO-.

Examples of the substituents in Z include halogen atoms (a fluorine atom, a chlorine atom, a bromine atom, an iodine atom), a carboxyl group, a C₂ to C₆ linear or branched acyl group, a C₁ to C₆ linear or branched alkyl group, and a C₁ to C₆ linear or branched alkoxy group.

Specific examples of the asymmetric acrylamide-methacrylic acid ester compound (A-2a) include, but are not particularly limited to, the following.

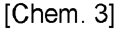

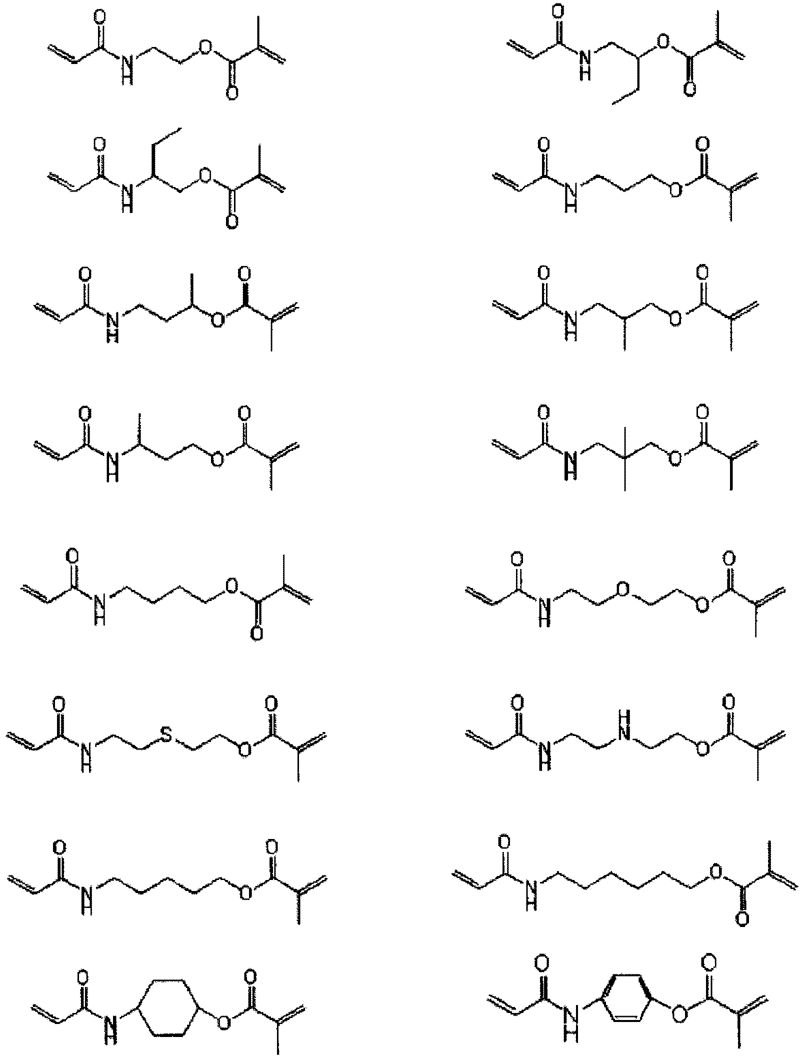

In view of adhesive properties to tooth structure and polymerization curability, preferred are asymmetric acrylamide-methacrylic acid ester compounds in which Z is an optionally substituted C₂ to C₄ linear or branched aliphatic group, more preferably N-methacryloyloxyethylacrylamide (commonly known as "MAEA"), N-methacryloyloxypropylacrylamide, N-methacryloyloxybutylacrylamide, N-(1-ethyl-(2-methacryloyloxy)ethyl)acrylamide, and N-(2-(2-methacryloyloxyethoxy)ethyl)acrylamide. In view of the high hydrophilicity concerning penetration into the collagen layer of dentin, most preferred are MAEA, and N-methacryloyloxypropylacrylamide.

The asymmetric acrylamide-methacrylic acid ester compound (A-2a) may be contained alone, or two or more thereof may be contained in combination. The content of asymmetric acrylamide-methacrylic acid ester compound (A-2a) is not particularly limited, as long as the present invention can exhibit its effects. However, the content of asymmetric acrylamide-methacrylic acid ester compound (A-2a) is preferably 1 to 60 parts by mass, more preferably 2 to 45 parts by mass, even more preferably 3 to 30 parts by mass, particularly preferably 5 to 25 parts by mass in total 100 parts by mass of the polymerizable monomer (A) in a composition for dental attachments of the present invention.

### Hydrophobic Polymerizable Monomer (A-2b) Having No Acidic Group

The hydrophobic polymerizable monomer (A-2b) having no acidic group (hereinafter, also referred to simply as "hydrophobic polymerizable monomer (A-2b)") improves properties such as ease of handling of the composition for dental attachments, and the mechanical strength of the cured product. The hydrophobic polymerizable monomer (A-2b) is preferably a radical polymerizable monomer having no acidic group but having a polymerizable group. In view of ease of radical polymerization, preferred as the polymerizable group are a (meth)acryl group and/or a (meth)acrylamide group. The hydrophobic polymerizable monomer (A-2b) means a polymerizable monomer that has no acidic group, and does not correspond to the asymmetric acrylamide-methacrylic acid ester compound (A-2a), and that has a solubility of less than 10 mass% in 25°C water. Examples of the hydrophobic polymerizable monomer (A-2b) include crosslinkable polymerizable monomers, such as aromatic bifunctional polymerizable monomers, aliphatic bifunctional polymerizable monomers, and tri- and higher-functional polymerizable monomers.

Examples of the aromatic bifunctional polymerizable monomers include 2,2-bis((meth)acryloyloxyphenyl)propane, 2,2-bis[4-(3-(meth)acryloyloxy-2-hydroxypropoxy)phenyl]propane, 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, and 2,2-bis(4-(meth)acryloyloxyisopropoxyphenyl)propane. Preferred are 2,2-bis[4-(3-methacryloyloxy-2-hydroxypropoxy)phenyl]propane (commonly known as "Bis-GMA"), 2,2-bis(4-(meth)acryloyloxyethoxyphenyl)propane, 2,2-bis(4-methacryloyloxypolyethoxyphenyl)propane (average number of moles of ethoxy group added: 2.6, commonly known as "D-2.6E"), 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, and 2,2-bis(4-(meth)acryloyloxypentaethoxyphenyl)propane.

Examples of the aliphatic bifunctional polymerizable monomers include glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, 1,3-butanediol di(meth)acrylate, 1,5-pentanediol di(meth)acrylate, 1,6-hexanediol di(meth)acrylate, 1,10-decanediol di(meth)acrylate, 1,2-bis(3-methacryloyloxy-2-hydroxypropoxy)ethane, and 2,2,4-trimethylhexamethylene bis(2-carbamoyloxyethyl)di(meth)acrylate. Preferred are triethylene glycol diacrylate, triethylene glycol dimethacrylate (commonly known as "3G"), neopentyl glycol di(meth)acrylate, 1,2-bis(3-methacryloyloxy-2-hydroxypropoxy)ethane, 2,2,4-trimethylhexamethylene bis(2-carbamoyloxyethyl)dimethacrylate (commonly known as "UDMA"), 1,10-decanediol dimethacrylate (commonly known as "DD"), and 2,2,4-trimethylhexamethylene bis(2-carbamoyloxyethyl)dimethacrylate.

Examples of the tri- and higher-functional polymerizable monomers include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol penta(meth)acrylate, N,N-(2,2,4-trimethylhexamethylene)bis[2-(aminocarboxy)propane-1,3-diol]tetra(meth)acrylate, and 1,7-diacryloyloxy-2,2,6,6-tetra(meth)acryloyloxymethyl-4-oxaheptane. Preferred is N,N-(2,2,4-trimethylhexamethylene)bis[2-(aminocarboxy)propane-1,3-diol]tetramethacrylate.

In view of mechanical strength and ease of handling, the preferred hydrophobic polymerizable monomers are aromatic bifunctional polymerizable monomers, and aliphatic bifunctional polymerizable monomers. Preferred as the aromatic bifunctional polymerizable monomers are Bis-GMA and D-2.6E. Preferred as the aliphatic bifunctional polymerizable monomers are glycerol di(meth)acrylate, 3G, neopentyl glycol di(meth)acrylate, 1,6-hexanediol di(meth)acrylate, DD, 1,2-bis(3-methacryloyloxy-2-hydroxypropoxy)ethane, and UDMA.

In view of the desirable adhesive properties for tooth structure of when the composition is used as a composition for dental attachments, the hydrophobic polymerizable monomer (A-2b) is more preferably Bis-GMA, D-2.6E, 3G, UDMA, or DD, even more preferably D-2.6E, 3G, or Bis-GMA.

The hydrophobic polymerizable monomer (A-2b) may be contained alone, or two or more thereof may be used in combination. The content of the hydrophobic polymerizable monomer (A-2b) in a composition for dental attachments of the present invention is preferably 20 to 99 parts by mass, more preferably 40 to 95 parts by mass, even more preferably 60 to 95 parts by mass in total 100 parts by mass of polymerizable monomer (A). By confining the content of hydrophobic polymerizable monomer (A-2b) in these ranges, sufficient adhesive properties can be obtained without reducing the wettability of the composition for dental attachments to tooth structure, and a sufficient strength can be obtained in the cured product.

### Hydrophilic Polymerizable Monomer (A-2c) Having No Acidic Group

In a composition for dental attachments of the present invention, the polymerizable monomer (A) preferably comprises a hydrophilic polymerizable monomer (A-2c) having no acidic group (hereinafter, also referred to simply as "hydrophilic polymerizable monomer (A-2c)"). The hydrophilic polymerizable monomer (A-2c) improves the wettability of the composition for dental attachments to tooth structure. The hydrophilic polymerizable monomer (A-2c) is preferably a radical polymerizable monomer having no acidic group but having a polymerizable group. In view of ease of radical polymerization, preferred as the polymerizable group are a (meth)acryl group and/or a (meth)acrylamide group. The hydrophilic polymerizable monomer (A-2c) means a polymerizable monomer that has no acidic group, and does not correspond to the asymmetric acrylamide-methacrylic acid ester compound (A-2a), and that has a solubility of 10 mass% or more in 25°C water. The polymerizable monomer is preferably one having a solubility of 30 mass% or more in 25°C water, more preferably one that can dissolve in water in any proportions at 25°C. The hydrophilic polymerizable monomer (A-2c) is preferably one having a hydrophilic group such as a hydroxyl group, an oxymethylene group, an oxyethylene group, an oxypropylene group, or an amide group. Examples of the hydrophilic polymerizable monomer (A-2c) include hydrophilic monofunctional (meth)acrylate polymerizable monomers such as 2-hydroxyethyl (meth)acrylate, 3-hydroxypropyl (meth)acrylate, 2-hydroxypropyl (meth)acrylate, 1,3-dihydroxypropyl (meth)acrylate, 2,3-dihydroxypropyl (meth)acrylate, 2-((meth)acryloyloxy)ethyltrimethyl ammonium chloride, and polyethylene glycol di(meth)acrylate (with nine or more oxyethylene groups); and hydrophilic monofunctional (meth)acrylamide polymerizable monomers such as N-methylol(meth)acrylamide, N-hydroxyethyl(meth)acrylamide, N,N-bis(2-hydroxyethyl)(meth)acrylamide, N-methoxymethyl(meth)acrylamide, N-ethoxymethyl(meth)acrylamide, diacetone(meth)acrylamide, 4-(meth)acryloylmorpholine, N-trihydroxymethyl-N-methyl(meth)acrylamide, N,N-dimethylacrylamide, and N,N-diethylacrylamide.

In view of adhesive properties to tooth structure, preferred as hydrophilic polymerizable monomer (A-2c) are 2-hydroxyethyl (meth)acrylate, 2,3-dihydroxypropyl (meth)acrylate, and hydrophilic monofunctional (meth)acrylamide polymerizable monomers, more preferably 2-hydroxyethyl (meth)acrylate, N,N-dimethylacrylamide, and N,N-diethylacrylamide. The hydrophilic polymerizable monomer (A-2c) may be contained alone, or two or more thereof may be used in combination.

The effect to improve adhesive properties may become insufficient when the content of the hydrophilic polymerizable monomer (A-2c) in a composition for dental attachments of the present invention is too low, whereas the mechanical strength of the cured product may decrease with an excessively high content of hydrophilic polymerizable monomer (A-2c). Accordingly, the content of the hydrophilic polymerizable monomer (A-2c) in a composition for dental attachments of the present invention preferably ranges from 0 to 50 parts by mass, more preferably 0 to 40 parts by mass, even more preferably 0 to 30 parts by mass in 100 parts by mass of polymerizable monomer (A). The content of hydrophilic polymerizable monomer (A-2c) may be 0 part by mass in 100 parts by mass of polymerizable monomer (A).

The content of polymerizable monomer (A-2) having no acidic group is preferably 50 to 99 parts by mass, more preferably 60 to 97 parts by mass, even more preferably 70 to 95 parts by mass in total 100 parts by mass of polymerizable monomer (A). In view of adhesive properties to uncut enamel after etching, the hydrophilic polymerizable monomer (A-2c) and the hydrophobic polymerizable monomer (A-2b) have a mass ratio of preferably 0:10 to 2:1, more preferably 0:10 to 1:1, even more preferably 0:10 to 1:2 in terms of a mass ratio of hydrophilic polymerizable monomer (A-2c) to hydrophobic polymerizable monomer (A-2b). In certain embodiments, it is preferable to contain 1 to 40 parts by mass of polymerizable monomer (A-1) having an acidic group, and 60 to 99 parts by mass of polymerizable monomer (A-2) having no acidic group, more preferably 2.5 to 35 parts by mass of polymerizable monomer (A-1) having an acidic group, and 65 to 97.5 parts by mass of polymerizable monomer (A-2) having no acidic group, even more preferably 5 to 30 parts by mass of polymerizable monomer (A-1) having an acidic group, and 70 to 95 parts by mass of polymerizable monomer (A-2) having no acidic group in total 100 parts by mass of polymerizable monomer (A).

A certain preferred embodiment is, for example, a composition for dental attachments that is essentially free of a bi- or higher-functional (meth)acrylamide polymerizable monomer. Another preferred embodiment, is, for example, a composition for dental attachment that is essentially free of a tri- or higher-functional (meth)acrylamide polymerizable monomer. Yet another preferred embodiment is, for example, a composition for dental attachment that is essentially free of a hydrogen phosphate diester group-containing polymerizable monomer. The hydrogen phosphate diester group-containing polymerizable monomer has a (meth)acryloyloxy group and/or a (meth)acrylamide group. In the present invention, "being essentially free of a polymerizable compound" means that the content of the polymerizable compound is less than 0.5 parts by mass, preferably less than 0.1 parts by mass, more preferably less than 0.01 parts by mass in total 100 parts by mass of the polymerizable monomers contained in the composition, and the content of the polymerizable compound may be 0 part by mass. The content of the polymerizable compound may be less than 0.5 mass%, or less than 0.1 mass% of the total composition.

Another certain preferred embodiment is, for example, a composition for dental attachments that is essentially free of a (meth)acrylic block copolymer. The (meth)acrylic block copolymer may have a molecular weight distribution (weight-average molecular weight/number average molecular weight) of, for example, 1.02 to 2.00. The molecular weight distribution can be measured by a known method, for example, gel permeation chromatography (GPC), and is calculated as a value in terms of standard polystyrene. The (meth)acrylic block copolymer may be bifunctional or higher functional, or may be tetrafunctional or higher functional.

### Photopolymerization Initiator (B)

The photopolymerization initiator (B) can be classified into a water-soluble photopolymerization initiator (B-1) and a water-insoluble photopolymerization initiator (B-2). The photopolymerization initiator (B) may be solely a water-soluble photopolymerization initiator (B-1) or a water-insoluble photopolymerization initiator (B-2), or may be a combination of a water-soluble photopolymerization initiator (B-1) and a water-insoluble photopolymerization initiator (B-2). Preferably, a water-soluble photopolymerization initiator (B-1) and a water-insoluble photopolymerization initiator (B-2) are used in combination.

### Water-Soluble Photopolymerization Initiator (B-1)

With a water-soluble photopolymerization initiator (B-1), a high bond strength can be achieved by improving polymerization curability at the interface with hydrophilic tooth surfaces. With the photopolymerization initiator (B) comprising a water-soluble photopolymerization initiator (B-1), the adhesive properties to uncut enamel after etching with phosphoric acid or the like can improve even more greatly. The water-soluble photopolymerization initiator (B-1) has a solubility in 25°C water of 10 g/L or more, preferably 15 g/L or more, more preferably 20 g/L or more, even more preferably 25 g/L or more. By having a solubility of 10 g/L or more in 25°C water, the water-soluble photopolymerization initiator (B-1) can sufficiently dissolve in water in tooth structure at the bond interface, and more easily develops the polymerization promoting effect.

Examples of the water-soluble photopolymerization initiator (B-1) include water-soluble acylphosphine oxides, water-soluble thioxanthones, α-hydroxyalkylacetophenones, and quaternary ammonium compounds prepared by quaternization of the amino group of α-aminoalkylphenones. The α-hydroxyalkylacetophenones may be, for example, compounds having a (poly)ethylene glycol chain introduced into the hydroxyl groups of 1-[4-(2-hydroxyethoxy)-phenyl]-2-hydroxy-2-methyl-1-propan-1-one, compounds having a (poly)ethylene glycol chain introduced into the hydroxyl group and/or phenyl group of 1-hydroxycyclohexyl phenyl ketone, compounds having -OCH₂COO⁻Na⁺ introduced into the phenyl group of 1-hydroxycyclohexyl phenyl ketone, compounds having a (poly)ethylene glycol chain introduced into the hydroxyl group and/or phenyl group of 2-hydroxy-2-methyl-1-phenylpropan-1-one, and compounds having -OCH₂COO⁻Na⁺ introduced into the phenyl group of 2-hydroxy-2-methyl-1-phenylpropan-1-one. The quaternary ammonium compounds, may be, for example, 2-methyl-1[4-(methylthio)phenyl]-2-morpholinopropan-1-one, and 2-benzyl-2-(dimethylamino)-1-[(4-morpholino)phenyl]-1-butanon.

The water-soluble thioxanthones may be any of, for example, 2-hydroxy-3-(9-oxo-9H-thioxanthen-4-yloxy)-N,N,N-trimethyl-1-propaneaminium chloride, 2-hydroxy-3-(1-methyl-9-oxo-9H-thioxanthen-4-yloxy)-N,N,N-trimethyl-1-propaneaminium chloride, 2-hydroxy-3-(9-oxo-9H-thioxanthen-2-yloxy)-N,N,N-trimethyl-1-propaneaminium chloride, 2-hydroxy-3-(3,4-dimethyl-9-oxo-9H-thioxanthen-2-yloxy)-N, N, N-trimethyl-1-propaneaminium chloride, 2-hydroxy-3-(3,4-dimethyl-9H-thioxanthen-2-yloxy)-N,N,N-trimethyl-1-propaneaminium chloride, and 2-hydroxy-3-(1,3,4-trimethyl-9-oxo-9H-thioxanthen-2-yloxy)-N,N,N-trimethyl-1-propaneaminium chloride.

Examples of the water-soluble acylphosphine oxides include acylphosphine oxides represented by the following general formula (2) or (3).

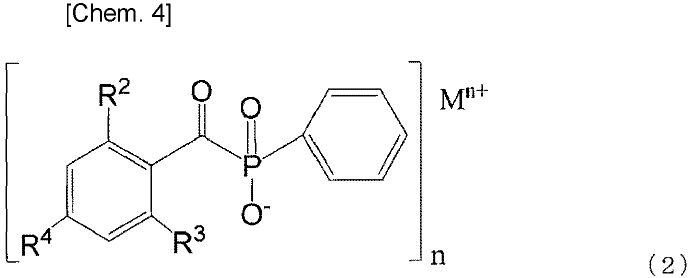

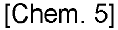

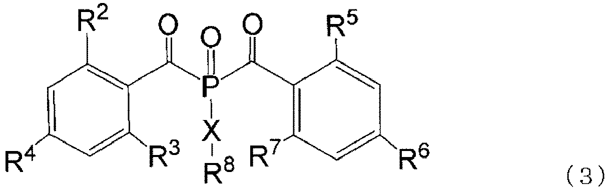

In formulae (2) and (3), R², R³, R⁴, R⁵, R⁶, and R⁷ are each independently a C₁ to C₄ linear or branched alkyl group or a halogen atom. In formula (2), M is a hydrogen ion, an alkali metal ion, an alkali earth metal ion, a magnesium ion, a pyridinium ion (the pyridine ring may have a substituent), or an ammonium ion represented by HN⁺R⁹R¹⁰R¹¹ (where R⁹, R¹⁰, and R¹¹ are each independently an organic group or a hydrogen atom), and n is 1 or 2. In formula (3), X is a C₁ to C₄ linear or branched alkylene group, R⁸ represents -CH(CH₃)COO(C₂H₄O)_{P}CH₃, where p represents an integer of 1 to 1,000.

The alkyl group represented by R², R³, R⁴, R⁵, R⁶, and R⁷ is not particularly limited, as long as it is a C₁ to C₄ linear or branched alkyl group. Examples include methyl, ethyl, n-propyl, isopropyl, n-butyl, isobutyl, sec-butyl, 2-methylpropyl, and tert-butyl. The alkyl group represented by R², R³, R⁴, R⁵, R⁶, and R⁷ is preferably a C₁ to C₃ linear alkyl group, more preferably methyl or ethyl, even more preferably methyl. Examples of the alkylene group represented by X include methylene, ethylene, n-propylene, isopropylene, and n-butylene. The alkylene group represented by X is preferably a C₁ to C₃ linear alkylene group, more preferably methylene or ethylene , even more preferably methylene.

Examples of the substituent of the pyridine ring when M is a pyridinium ion include halogen atoms (a fluorine atom, a chlorine atom, a bromine atom, an iodine atom), a carboxyl group, a C₂ to C₆ linear or branched acyl group, a C₁ to C₆ linear or branched alkyl group, and a C₁ to C₆ linear or branched alkoxy group. Preferably, M is an alkali metal ion, an alkali earth metal ion, a magnesium ion, a pyridinium ion (the pyridine ring may have a substituent), or an ammonium ion represented by HN⁺R⁹R¹⁰R¹¹ (the symbols have the same meaning as above). Examples of the alkali metal ion include a lithium ion, a sodium ion, a potassium ion, a rubidium ion, and a cesium ion. Examples of the alkali earth metal ion include a calcium ion, a strontium ion, a barium ion, and a radium ion. The organic group represented by R⁹, R¹⁰, and R¹¹ may be the same group exemplified above for the substituent of the pyridine ring (excluding a halogen atom).

In view of storage stability and shade stability in the composition, particularly preferred are compounds in which R², R³, R⁴, R⁵, R⁶, and R⁷ in formulae (2) and (3) are all methyl groups. Examples of Mⁿ⁺ include Li⁺, Na⁺, K⁺, Ca²⁺, Mg²⁺, and ammonium ions derived from various amines. Examples of the amines include ammonia, trimethylamine, diethylamine, dimethylaniline, ethylenediamine, triethanolamine, N,N-dimethylaminomethacrylate, 4-(N,N-dimethylamino)benzoic acid and alkyl esters thereof, 4-(N,N-diethylamino)benzoic acid and alkyl esters thereof, and N,N-bis(2-hydroxyethyl)-p-toluidine. In view of adhesive properties, p in R⁸ is preferably 1 or more, more preferably 2 or more, even more preferably 3 or more, particularly preferably 4 or more, and is preferably 1,000 or less, more preferably 100 or less, even more preferably 75 or less, particularly preferably 50 or less.

Particularly preferably, the water-soluble acylphosphine oxides are compounds represented by general formula (2) and in which M⁺ is Li⁺, and compounds represented by general formula (3) and in which the moiety corresponding to the group represented by R⁸ is synthesized from polyethylene glycol methylether methacrylate having a molecular weight of 950. In these compounds, R², R³, and R⁴ in general formula (2), and R², R³, R⁴, R⁵, R⁶, and R⁷ in general formula (3) are the same as above.

The water-soluble acylphosphine oxides having such structures can be synthesized according to known methods, and some are available as commercially available products. For example, the methods disclosed in JP S57-197289 A and WO2014/095724 can be used for the synthesis of the water-soluble acylphosphine oxides. The water-soluble photopolymerization initiator (B-1) may be used alone, or two or more thereof may be used in combination.

The water-soluble photopolymerization initiator (B-1) may be dissolved in the composition for dental attachments, or may be dispersed in the form of a powder in the composition.

When the water-soluble photopolymerization initiator (B-1) is dispersed in the form of a powder, the average particle diameter is preferably 500 µm or less, more preferably 100 µm or less, even more preferably 50 µm or less because the water-soluble photopolymerization initiator (B-1) tends to precipitate when the average particle diameter is excessively large. The average particle diameter is preferably 0.01 µm or more because the specific surface area of the powder overly increases, and the amount of powder that can be dispersed in the composition decreases when the average particle diameter is excessively small. Taken together, the average particle diameter of water-soluble photopolymerization initiator (B-1) preferably ranges from 0.01 to 500 µm, more preferably 0.01 to 100 µm, even more preferably 0.01 to 50 µm.

The average particle diameter of a powder of water-soluble photopolymerization initiator (B-1) can be determined by taking an electron micrograph of at least 100 particles, and calculating the volume average particle diameter from the captured image after an image analysis performed with image-analyzing particle size distribution measurement software (Mac-View, manufactured by Mountech Co., Ltd.).

The shape of the water-soluble photopolymerization initiator (B-1) when it is dispersed in the form of a powder is not particularly limited, and may be any of various shapes, including, for example, spherical, stylus, plate-like, and crushed shapes. The water-soluble photopolymerization initiator (B-1) can be prepared using a known method such as pulverization, freeze drying, or reprecipitation. In view of the average particle diameter of the powder obtained, freeze drying and reprecipitation are preferred, and freeze drying is more preferred.

In view of curability and other properties of the composition for dental attachments obtained, the content of water-soluble photopolymerization initiator (B-1) is preferably 0.01 to 20 parts by mass relative to total 100 parts by mass of the polymerizable monomer (A) in a composition for dental attachments of the present invention. In view of adhesive properties to tooth structure, the content of water-soluble photopolymerization initiator (B-1) is more preferably 0.05 to 10 parts by mass, even more preferably 0.1 to 5 parts by mass relative to total 100 parts by mass of the polymerizable monomer (A) in a composition for dental attachments of the present invention. When the content of water-soluble photopolymerization initiator (B-1) is less than 0.01 parts by mass, polymerization may fail to sufficiently proceed at the bond interface, and the bond strength may decrease. When the content of water-soluble photopolymerization initiator (B-1) is more than 20 parts by mass, it may not be possible to obtain a sufficient bond strength, in addition to making it difficult to sufficiently dissolve, disperse, or diffuse water-soluble photopolymerization initiator (B-1) in the composition for dental attachments.

### Water-Insoluble Photopolymerization Initiator (B-2)

In view of curability, a composition for dental attachments of the present invention preferably comprises a water-insoluble photopolymerization initiator (B-2) having a solubility of less than 10 g/L in 25°C water (hereinafter, also referred to as "water-insoluble photopolymerization initiator (B-2)"). The water-insoluble photopolymerization initiator (B-2) used in the present invention may use a known photopolymerization initiator. The water-insoluble photopolymerization initiator (B-2) may be contained alone, or two or more thereof may be contained in combination.

Examples of the water-insoluble photopolymerization initiator (B-2) include (bis)acylphosphine oxides (other than those exemplified for water-soluble photopolymerization initiator (B-1)), thioxanthones, ketals, α-diketones, coumarins, anthraquinones, benzoin alkyl ether compounds, and α-aminoketone compounds.

Examples of acylphosphine oxides in the (bis)acylphosphine oxides include 2,4,6-trimethylbenzoyldiphenylphosphine oxide, 2,6-dimethoxybenzoyldiphenylphosphine oxide, 2,6-dichlorobenzoyldiphenylphosphine oxide, 2,4,6-trimethylbenzoylmethoxyphenylphosphine oxide, 2,4,6-trimethylbenzoylethoxyphenylphosphine oxide, 2,3,5,6-tetramethylbenzoyldiphenylphosphine oxide, and benzoyl di(2,6-dimethylphenyl)phosphonate. Examples of bisacylphosphine oxides include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, and bis(2,5,6-trimethylbenzoyl)-2,4,4-trimethylpentylphosphine oxide.

Examples of the thioxanthones include thioxanthone, and 2-chlorothioxanthen-9-one.

Examples of the ketals include benzyl dimethyl ketal, and benzyl diethyl ketal.

Examples of the α-diketones include diacetyl, benzyl, dl-camphorquinone, 2,3-pentadione, 2,3-octadione, 9,10-phenanthrenequinone, 4,4'-oxybenzyl, and acenaphthenequinone. Particularly preferred is dl-camphorquinone for its maximum absorption wavelength occurring in the visible light region.

Examples of the coumarins include compounds mentioned in JP H9-3109 A and JP H10-245525A, including, for example, 3,3'-carbonylbis(7-diethylaminocoumarin), 3-(4-methoxybenzoyl)coumarin, 3-thienoylcoumarin, 3-benzoyl-5,7-dimethoxycoumarin, 3-benzoyl-7-methoxycoumarin, 3-benzoyl-6-methoxycoumarin, 3-benzoyl-8-methoxycoumarin, 3-benzoylcoumarin, 7-methoxy-3-(p-nitrobenzoyl)coumarin, 3-(p-nitrobenzoyl)coumarin, 3,5-carbonylbis(7-methoxycoumarin), 3-benzoyl-6-bromocoumarin, 3,3'-carbonylbiscoumarin, 3-benzoyl-7-dimethylaminocoumarin, 3-benzoylbenzo[f]coumarin, 3-carboxycoumarin, 3-carboxy-7-methoxycoumarin, 3-ethoxycarbonyl-6-methoxycoumarin, 3-ethoxycarbonyl-8-methoxycoumarin, 3-acetylbenzo[f]coumarin, 3-benzoyl-6-nitrocoumarin, 3-benzoyl-7-diethylaminocoumarin, 7-dimethylamino-3-(4-methoxybenzoyl)coumarin, 7-diethylamino-3-(4-methoxybenzoyl)coumarin, 7-diethylamino-3-(4-diethylamino)coumarin, 7-methoxy-3-(4-methoxybenzoyl)coumarin, 3-(4-nitrobenzoyl)benzo[f]coumarin, 3-(4-ethoxycinnamoyl)-7-methoxycoumarin, 3-(4-dimethylaminocinnamoyl)coumarin, 3-(4-diphenylaminocinnamoyl)coumarin, 3-[(3-dimethylbenzothiazol-2-ylidene)acetyl]coumarin, 3-[(1-methylnaphtho[1,2-d]thiazol-2-ylidene)acetyl]coumarin, 3,3'-carbonylbis(6-methoxycoumarin), 3,3'-carbonylbis(7-acetoxycoumarin), 3,3'-carbonylbis(7-dimethylaminocoumarin), 3-(2-benzothiazolyl)-7-(diethylamino)coumarin, 3-(2-benzothiazolyl)-7-(dibutylamino)coumarin, 3-(2-benzoimidazolyl)-7-(diethylamino)coumarin, 3-(2-benzothiazolyl)-7-(dioctylamino)coumarin, 3-acetyl-7-(dimethylamino)coumarin, 3,3'-carbonylbis(7-dibutylaminocoumarin), 3,3'-carbonyl-7-diethylaminocoumarin-7'-bis(butoxyethyl)aminocoumarin, 10-[3-[4-(dimethylamino)phenyl]-1-oxo-2-propenyl]-2,3,6,7-tetrahydro-1,1,7,7-tetramethyl-1H,5H,11H-[1]benzopyrrano[6,7,8-ij]quinolizin-11 -one, and 10-(2-benzothiazolyl)-2,3,6,7-tetrahydro-1,1,7,7-tetramethyl-1H,5H,11H-[1]benzopyrrano[6,7,8-ij]quinolizin-11-one.

Particularly preferred among these coumarins are 3,3'-carbonylbis(7-diethylaminocoumarin) and 3,3'-carbonylbis(7-dibutylaminocoumarin).

Examples of the anthraquinones include anthraquinone, 1-chloroanthraquinone, 2-chloroanthraquinone, 1-bromoanthraquinone, 1,2-benzanthraquinone, 1-methylanthraquinone, 2-ethylanthraquinone, and 1-hydroxyanthraquinone.

Example of the benzoin alkyl ether compounds include benzoin methyl ether, benzoin ethyl ether, benzoin isopropyl ether, and benzoin isobutyl ether.

Examples of the α-aminoketone compounds include 2-methyl-1-[4-(methylthio)phenyl]-2-morpholinopropan-1-one.

The water-insoluble photopolymerization initiator (B-2) is preferably at least one selected from the group consisting of a (bis)acylphosphine oxide, an α-diketone, and a coumarin. In this way, a composition for dental attachments can be obtained that has excellent photocurability both in the visible light region and the near ultraviolet region, and that shows sufficient photocurability regardless of whether the light source used is a halogen lamp, a light emitting diode (LED), or a xenon lamp.

The content of water-insoluble photopolymerization initiator (B-2) is not particularly limited. However, in view of curability and other properties of the composition obtained, the content of water-insoluble photopolymerization initiator (B-2) is preferably 0.01 to 10 parts by mass, more preferably 0.05 to 7 parts by mass, even more preferably 0.1 to 5 parts by mass relative to total 100 parts by mass of the polymerizable monomer (A) in a composition for dental attachments of the present invention. When the content of water-insoluble photopolymerization initiator (B-2) is more than 10 parts by mass, it may not be possible to obtain a sufficient bond strength when the polymerization initiator itself has low polymerization performance, in addition to raising a possibility of precipitation from the composition for dental attachments.

When the water-soluble photopolymerization initiator (B-1) and the water-insoluble photopolymerization initiator (B-2) are used in combination, the mass ratio (B-1):(B-2) of the water-soluble photopolymerization initiator (B-1) to the water-insoluble photopolymerization initiator (B-2) in the present invention is preferably 10:1 to 1:10, more preferably 7:1 to 1:7, even more preferably 5:1 to 1:5, most preferably 3:1 to 1:3. When the fraction of water-soluble photopolymerization initiator (B-1) in the mass ratio exceeds 10:1, the curability of the composition for dental attachments itself decreases, and the composition may have difficulty in exhibiting high bond strength. When the fraction of water-insoluble photopolymerization initiator (B-2) in the mass ratio exceeds 1:10, the composition for dental attachments may have difficulty in exhibiting high bond strength as a result of insufficient promotion of polymerization at the bond interface, though the curability of the composition itself can increase.

### Filler (C)

A composition for dental attachments of the present invention must comprise a filler (C), in order to adjust ease of handling, and to increase the mechanical strength of the cured product. Examples of such fillers include inorganic fillers and organic-inorganic composite fillers. Examples of the materials of organic fillers include polymethylmethacrylate, polyethylmethacrylate, a methyl methacrylate-ethyl methacrylate copolymer, crosslinked polymethylmethacrylate, crosslinked polyethylmethacrylate, polyamides, polyvinyl chloride, polystyrene, chloroprene rubber, nitrile rubber, an ethylene-vinyl acetate copolymer, a styrene-butadiene copolymer, an acrylonitrile-styrene copolymer, and an acrylonitrile-styrene-butadiene copolymer. These may be used alone, or two or more thereof may be used as a mixture. The shape of organic filler is not particularly limited, and the particle diameter of the filler may be appropriately selected for use. In view of considerations such as the ease of handling and mechanical strength of the composition for dental attachments obtained, the average particle diameter of the organic filler is preferably 0.001 to 50 µm, more preferably 0.001 to 10 µm.

Examples of the materials of the inorganic fillers include quartz, silica, alumina, silica-titania, silica-titania-barium oxide, silica-zirconia, silica-alumina, lanthanum glass, borosilicate glass, soda glass, barium glass, strontium glass, glass-ceramics, aluminosilicate glass, barium boroaluminosilicate glass, strontium boroaluminosilicate glass, fluoroaluminosilicate glass, calcium fluoroaluminosilicate glass, strontium fluoroaluminosilicate glass, barium fluoroaluminosilicate glass, strontium calcium fluoroaluminosilicate glass, ytterbium oxide, and silica-coated ytterbium fluoride. These may be used alone, or two or more thereof may be used as a mixture. The shape of inorganic filler is not particularly limited, and the particle diameter of the filler may be appropriately selected for use. For considerations such as superiority of the mechanical strength and transparency of the composition for dental attachments obtained, preferred for use are quartz, silica, silica-zirconia, barium glass, ytterbium oxide, and silica-coated ytterbium fluoride, more preferably quartz, silica, silica-zirconia, barium glass, and silica-coated ytterbium fluoride. In view of considerations such as the ease of handling and mechanical strength of the composition for dental attachments obtained, the average particle diameter of the inorganic filler is preferably 0.001 to 50 µm, more preferably 0.001 to 10 µm. In this specification, the average particle diameter of inorganic filler means the average particle diameter before surface treatment when the inorganic filler is subjected to a surface treatment as will be described. A certain preferred embodiment is, for example, a composition for dental attachments in which the filler (C) is an inorganic filler.

The inorganic filler may be, for example, an irregularly shaped filler or a spherical filler. In view of improving the mechanical strength of a cured product of the composition for dental attachments, the inorganic filler used is preferably a spherical filler. Here, the spherical filler is a filler having an average uniformity of 0.6 or more as measured for round-shaped particles observed in a unit field of an electron micrograph of filler by dividing a particle diameter along a direction orthogonal to the maximum diameter by the maximum diameter. The spherical filler has an average particle diameter of preferably 0.05 to 5 µm. When the average particle diameter is less than 0.05 µm, the mechanical strength may decrease as a result of a decrease of the filling rate of the spherical filler in the composition for dental attachments. When the average particle diameter is more than 5 µm, the spherical filler has a reduced surface area, and the composition for dental attachments may fail to produce a cured product having high mechanical strength.

In order to adjust the flowability of the composition for dental attachments, the inorganic filler may be used after an optional surface treatment with a known surface treatment agent such as a silane coupling agent. Examples of the surface treatment agent include vinyltrimethoxysilane, vinyltriethoxysilane, vinyltrichlorosilane, vinyl tri(β-methoxyethoxy)silane, γ-methacryloyloxypropyltrimethoxysilane, 8-methacryloyloxyoctyltrimethoxysilane, 11-methacryloyloxyundecyltrimethoxysilane, γ-glycidoxypropyltrimethoxysilane, γ-mercaptopropyltrimethoxysilane, and γ-aminopropyltriethoxysilane.

The surface treatment can be carried out using any known method, including, for example, a method that adds the surface treatment agent by spraying it while vigorously stirring the inorganic filler, a method that disperses or dissolves the inorganic filler and surface treatment agent in a suitable solvent, and removes the solvent, and a method in which the alkoxy group of the surface treatment agent is transformed into a silanol group by hydrolysis with an acid catalyst in an aqueous solution, and the silanol group is allowed to adhere to the inorganic filler surface in the aqueous solution before removal of water. In all of these methods, the reaction between the inorganic filler surface and the surface treatment agent can proceed to completion to enable a surface treatment by applying heat in a temperature range of typically 50 to 150°C. The amount of surface treatment agent is not particularly limited, and the surface treatment agent may be used in an amount of, for example, 1 to 10 parts by mass relative to 100 parts by mass of the inorganic filler before treatment.

The organic-inorganic composite filler used in the present invention is a filler obtained by adding a polymerizable monomer to the inorganic filler, polymerizing the mixture in paste form, and pulverizing the polymerized filler. The organic-inorganic composite filler may be, for example, a TMPT filler (a filler obtained by mixing trimethylolpropane methacrylate and a silica filler, and pulverizing the mixture after polymerization). The shape of the organic-inorganic composite filler is not particularly limited, and may be determined by appropriately selecting the particle size of the filler. In view of properties such as the ease of handling and mechanical strength of the composition obtained, the organic-inorganic composite filler has an average particle diameter of preferably 0.001 to 50 µm, more preferably 0.001 to 10 µm.

In this specification, the average particle diameter of filler can be determined by a laser diffraction scattering method or by observing particles with an electron microscope. Specifically, a laser diffraction scattering method is more convenient for the measurement of particles having a particle size of 0.1 µm or more, whereas electron microscopy is a more convenient method of particle size measurement for ultrafine particles of less than 0.1 µm. Here, 0.1 µm is a measured value by a laser diffraction scattering method.

As a specific example of a laser diffraction scattering method, the particle size may be measured by volume using, for example, a laser diffraction particle size distribution analyzer (SALD-2300, manufactured by Shimadzu Corporation) with a 0.2% sodium hexametaphosphate aqueous solution used as dispersion medium.

In electron microscopy, for example, particles may be photographed with an electron microscope (Model S-4000, manufactured by Hitachi), and the size of particles (at least 200 particles) observed in a unit field of the captured image may be measured using image-analyzing particle-size-distribution measurement software (Mac-View; Mountech Co., Ltd.). Here, the particle diameter is determined as an arithmetic mean value of the maximum and minimum lengths of particles, and the average primary particle diameter is calculated from the number of particles and the particle diameter.

In a composition for dental attachments of the present invention, it is preferable to use a mixture or a combination of two or more fillers of different materials, particle size distributions, and forms. By combining two or more types of fillers, the fillers can interact with the polymerizable monomer or with the other fillers at an increased number of points, in addition to densely filling the composition. This provides the appropriate flexural modulus and hardness needed for dental attachments. Depending on the type of filler, it is also possible to control paste fluidity in the presence or absence of a shear force. In view of the ease of handling and paste properties of a composition for dental attachments of the present invention, the filler (C) is preferably a combination (I) of a filler (C-1) having an average particle diameter of 1 nm or more and less than 0.1 µm, and a filler (C-2) having an average particle diameter of 0.1 µm or more and 1 µm or less; a combination (II) of a filler (C-1) having an average particle diameter of 1 nm or more and less than 0.1 µm, and a filler (C-3) having an average particle diameter of more than 1 µm and 10 µm or less; a combination (III) of a filler (C-1) having an average particle diameter of 1 nm or more and less than 0.1 µm, a filler (C-2) having an average particle diameter of 0.1 µm or more and 1 µm or less, and a filler (C-3) having an average particle diameter of more than 1 µm and 10 µm or less; or a combination (IV) of fillers (C-2) having an average particle diameter of 0.1 µm or more and 1 µm or less. The combinations (I), (II), and (III) are more preferred, and the combinations (I) and (II) are even more preferred because these combinations enable the cured product dental attachments to have more desirable flexural modulus, and exhibit stronger orthodontic forces as an orthodontic appliance jointly with an orthodontic aligner. Concerning the ease of handling of dental attachments, the composition requires the fluidity that enables the composition to easily fill an attachment template for the formation of dental attachments, and the formability that enables the composition to stay in position once the bonding locations are determined on tooth structure. Indeed, the properties required for handling are different from common dental composite resins, which require the fluidity to fill cavities, but do not require the level of formability required for dental attachments. The combination (IV) of fillers (C-2) having an average particle diameter of 0.1 µm or more and 1 µm or less means an embodiment that comprises two types of fillers (C-2) of different average particle diameters between 0.1 µm and 1 µm. The average particle diameter of filler (C-1) is preferably 1 nm or more and 90 nm or less, more preferably 2 nm or more and 80 nm or less, even more preferably 3 nm or more and 70 nm or less. The average particle diameter of filler (C-2) is preferably 0.1 µm or more and 0.9 µm or less, more preferably 0.15 µm or more and 0.85 µm or less, even more preferably 0.2 µm or more and 0.8 µm or less. The average particle diameter of filler (C-3) is preferably 1.2 µm or more and 9 µm or less, more preferably 1.5 µm or more and 8 µm or less, even more preferably 2.0 µm or more and 7 µm or less. The fillers (C) of different particle diameters may include other types of fillers, provided that the fillers (C) have the foregoing combinations. Unintended inclusion of non-filler particles as impurities is acceptable to such an extent that it does not hinder the effects of the present invention.

The content of filler (C) is not particularly limited. In view of the mechanical strength of the cured product and adhesive properties to uncut enamel, 50 to 90 parts by mass of filler (C) is required in total 100 parts by mass of the composition for dental attachments. Preferably, the content of filler (C) is 55 to 85 parts by mass, more preferably 60 to 80 parts by mass in total 100 parts by mass of the composition for dental attachments.

A composition for dental attachments of the present invention can be produced with ease using a method known to a person skilled in the art, and the method of production is not particularly limited, provided that the composition comprises a polymerizable monomer (A), a photopolymerization initiator (B), and a filler (C), and that the polymerizable monomer (A) comprises a polymerizable monomer (A-1) having an acidic group and a polymerizable monomer (A-2) having no acidic group, and the content of the polymerizable monomer (A-1) having an acidic group is 1 to 40 parts by mass in total 100 parts by mass of the polymerizable monomer (A), and the content of the filler (C) is 50 to 90 parts by mass in total 100 parts by mass of the composition for dental attachments.

### Polymerization Accelerator (D)

A composition for dental attachments of the present invention may use a polymerization accelerator (D) with the water-insoluble photopolymerization initiator (B-2) and/or a chemical polymerization initiator (described later). Examples of the polymerization accelerator (D) that can be used in the present invention include amines, sulfinic acid and salts thereof, borate compounds, derivatives of barbituric acid, triazine compounds, copper compounds, tin compounds, vanadium compounds, halogen compounds, aldehydes, thiol compounds, sulfites, bisulfites, and thiourea compounds.

The amines used as polymerization accelerator (D) can be categorized into aliphatic amines and aromatic amines. Examples of the aliphatic amines include primary aliphatic amines such as n-butylamine, n-hexylamine, and n-octylamine; secondary aliphatic amines such as diisopropylamine, dibutylamine, and N-methylethanolamine; and tertiary aliphatic amines such as N-methyldiethanolamine, N-ethyldiethanolamine, N-n-butyldiethanolamine, N-lauryldiethanolamine, 2-(dimethylamino)ethyl methacrylate, N-methyldiethanolamine dimethacrylate, N-ethyldiethanolamine dimethacrylate, triethanolamine monomethacrylate, triethanolamine dimethacrylate, triethanolamine trimethacrylate, triethanolamine, trimethylamine, triethylamine, and tributylamine. In view of the curability and storage stability of the composition for dental attachments, preferred for use are tertiary aliphatic amines, more preferably N-methyldiethanolamine and triethanolamine.

Examples of the aromatic amines include N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-diisopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, ethyl 4-(N,N-dimethylamino)benzoate, methyl 4-(N,N-dimethylamino)benzoate, propyl 4-(N,N-dimethylamino)benzoate, n-butoxyethyl 4-(N,N-dimethylamino)benzoate, 2-(methacryloyloxy)ethyl 4-(N,N-dimethylamino)benzoate, 4-(N,N-dimethylamino)benzophenone, and butyl 4-(N,N-dimethylamino)benzoate. In view of the ability to impart excellent curability to the composition for dental attachments, it is preferable to use at least one selected from the group consisting of N,N-bis(2-hydroxyethyl)-p-toluidine, ethyl 4-(N,N-dimethylamino)benzoate, n-butoxyethyl 4-(N,N-dimethylamino)benzoate, and 4-(N,N-dimethylamino)benzophenone.

Specific examples of sulfinic acid and salts thereof, borate compounds, derivatives of barbituric acid, triazine compounds, copper compounds, tin compounds, vanadium compounds, halogen compounds, aldehydes, thiol compounds, sulfites, bisulfites, and thiourea compounds include those mentioned in WO2008/087977.

The polymerization accelerator (D) may be contained alone, or two or more thereof may be contained in combination. The content of the polymerization accelerator (D) used in the present invention is not particularly limited. However, in view of the curability and other properties of the composition for dental attachments obtained, the content of polymerization accelerator (D) is preferably 0.001 to 30 parts by mass, more preferably 0.01 to 10 parts by mass, even more preferably 0.1 to 5 parts by mass relative to total 100 parts by mass of the polymerizable monomer (A) in the composition for dental attachments. When the content of polymerization accelerator (D) is less than 0.001 parts by mass, polymerization may fail to proceed sufficiently, and this may lead to a decrease of adhesive properties. In this respect, the content of polymerization accelerator (D) is more preferably 0.05 parts or more by mass. When the content of polymerization accelerator (D) is more than 30 parts by mass, it may not be possible to obtain sufficient adhesive properties, in addition to raising a possibility of precipitation from the composition for dental attachments. In this respect, the content of polymerization accelerator (D) is more preferably 20 parts or less by mass.

### Chemical Polymerization Initiator

A composition for dental attachments of the present invention may additionally comprise a chemical polymerization initiator. Preferred for use as chemical polymerization initiators are organic peroxides. The organic peroxide used as the chemical polymerization initiator is not particularly limited, and known organic peroxides may be used. Typical examples of such organic peroxides include ketoneperoxides, hydroperoxides, diacyl peroxides, dialkyl peroxides, peroxyketals, peroxyesters, and peroxy dicarbonates. Specific examples of these organic peroxides include those mentioned in WO2008/087977. The chemical polymerization initiator may be used alone, or two or more thereof may be used in combination.

### Fluorine-Ion Releasing Substance

A composition for dental attachments of the present invention may additionally comprise a fluorine-ion releasing substance. By containing a fluorine-ion releasing substance, the composition for dental attachments produced can impart acid resistance to tooth structure. Examples of the fluorine-ion releasing substance include metal fluorides such as sodium fluoride, potassium fluoride, sodium monofluorophosphate, lithium fluoride, and ytterbium fluoride. The fluorine-ion releasing substance may be contained alone, or two or more thereof may be contained in combination.

A composition for dental attachments of the present invention may also comprise a known additive, provided that such additives do not cause a performance drop. Examples of additives that may be contained in a composition for dental attachments of the present invention include polymerization inhibitors, antioxidants, colorants (pigments, dyes), ultraviolet absorbers, solvents such as water and organic solvents, and thickeners. The additives may be used alone, or two or more thereof may be used in combination. In certain embodiments, the content of the solvent (for example, water, organic solvent) in the composition for dental attachments is preferably less than 1 mass%, more preferably less than 0.1 mass%, even more preferably less than 0.01 mass% based on the total amount of the composition for dental attachments.

Examples of the polymerization inhibitors include hydroquinone, hydroquinone monomethyl ether, dibutylhydroquinone, dibutylhydroquinone monomethyl ether, t-butylcatechol, 2-t-butyl-4,6-dimethylphenol, 2,6-di-t-butylphenol, and 3,5-di-t-butyl-4-hydroxytoluene. The content of the polymerization inhibitor is preferably 0.001 to 1.0 parts by mass relative to 100 parts by mass of the polymerizable monomer (A) in the composition for dental attachments.

The composition for dental attachments exhibits excellent adhesive properties to enamel after phosphoric acid etching, without carrying out a pretreatment with, for example, a dental adhesive after etching with phosphoric acid or the like, and can simplify the bonding procedures. This makes the composition for dental attachments suited for use as a dental attachment for aligner orthodontics.

Examples of the fractions of the components in a composition for dental attachments suited as a dental attachment for aligner orthodontics are as follows. By taking the total amount of polymerizable monomer (A) as 100 parts by mass, the composition preferably comprises 1 to 40 parts by mass of polymerizable monomer (A-1) having an acidic group, and 60 to 99 parts by mass of polymerizable monomer (A-2) having no acidic group in 100 parts by mass of polymerizable monomer (A), and 0.05 to 10 parts by mass of photopolymerization initiator (B), 100 to 900 parts by mass of filler (C), and 0.001 to 30 parts by mass of polymerization accelerator (D) relative to 100 parts by mass of polymerizable monomer (A). More preferably, the composition comprises 2.5 to 35 parts by mass of polymerizable monomer (A-1) having an acidic group, and 65 to 97.5 parts by mass of polymerizable monomer (A-2) having no acidic group in 100 parts by mass of polymerizable monomer (A), and 0.1 to 5 parts by mass of photopolymerization initiator (B), 120 to 560 parts by mass of filler (C), and 0.01 to 10 parts by mass of polymerization accelerator (D) relative to 100 parts by mass of polymerizable monomer (A). Even more preferably, the composition comprises 5 to 30 parts by mass of polymerizable monomer (A-1) having an acidic group, and 70 to 95 parts by mass of polymerizable monomer (A-2) having no acidic group in 100 parts by mass of polymerizable monomer (A), and 0.15 to 2.5 parts by mass of photopolymerization initiator (B), 150 to 400 parts by mass of filler (C), and 0.1 to 5 parts by mass of polymerization accelerator (D) relative to 100 parts by mass of polymerizable monomer (A).

Another embodiment is, for example, an orthodontic kit comprising the composition for dental attachments, and an orthodontic aligner. The orthodontic aligner may be any known orthodontic aligner. The orthodontic aligner may be, for example, a photocurable composition comprising a polymerizable monomer, a photopolymerization initiator, and, optionally, a filler. The polymerizable monomer, photopolymerization initiator, and filler may use the polymerizable monomer (A), photopolymerization initiator (B), and filler (C).

The form of a composition for dental attachments of the present invention is not particularly limited, and may be, for example, a two-pack type (two-paste type). However, in view of ease of handling, a composition for dental attachments of the present invention is preferably of a one-pack type (one-paste type) in which all the components are premixed. More preferably, a composition for dental attachments of the present invention is used by being filled in a cylindrical syringe container. The cylindrical portion of the syringe container is preferably 10 cm in length and 15 mm or less in inner diameter, more preferably 7.5 cm in length and 10 mm or less in inner diameter. For improved ease of handling, the syringe may be used with a nozzle attached to the tip. The nozzle is preferably 25 mm long, and has an opening with an inside diameter of 1.5 mm or less. More preferably, the nozzle length is 20 mm, and the opening has an inside diameter of 0.75 mm or less.

A certain embodiment is, for example, a composition for dental attachments that comprises a polymerizable monomer (A), a photopolymerization initiator (B), and a filler (C), and in which the polymerizable monomer (A) comprises a polymerizable monomer (A-1) having an acidic group and a polymerizable monomer (A-2) having no acidic group, and the content of the polymerizable monomer (A-1) having an acidic group is 1 to 40 parts by mass in 100 parts by mass of the polymerizable monomer (A), and the content of the filler (C) is 50 to 90 parts by mass in total 100 parts by mass of the composition.

The use may be a use for forming a dental attachment. The use may be a use for securing an orthodontic aligner. The use may be a use on tooth surface. The use may be a nontherapeutic use. Another embodiment may be a use of the composition for dental attachments for orthodontic treatment. Another embodiment may be a use of the composition for dental attachments for the treatment of dental disease. Examples of the dental disease include jaw deformities, occlusal abnormalities, and congenital disorders (for example, cheilognathopalatoschisis, cleidocranial dysplasia, Pierre Robin syndrome, and branchial arch syndrome).

Another certain embodiment is, for example, a method of using a composition for dental attachments for tooth surface for the formation of a dental attachment on tooth surface, wherein the composition for dental attachments comprises a polymerizable monomer (A), a photopolymerization initiator (B), and a filler (C), in which the polymerizable monomer (A) comprises a polymerizable monomer (A-1) having an acidic group and a polymerizable monomer (A-2) having no acidic group, and the content of the polymerizable monomer (A-1) having an acidic group is 1 to 40 parts by mass in 100 parts by mass of the polymerizable monomer (A), and the content of the filler (C) is 50 to 90 parts by mass in total 100 parts by mass of the composition. Another certain embodiment is, for example, a method of production of a dental attachment on tooth surface, wherein the dental attachment is a cured product of a composition for dental attachments, and in which the composition for dental attachments comprises a polymerizable monomer (A), a photopolymerization initiator (B), and a filler (C), and the polymerizable monomer (A) comprises a polymerizable monomer (A-1) having an acidic group and a polymerizable monomer (A-2) having no acidic group, and the content of the polymerizable monomer (A-1) having an acidic group is 1 to 40 parts by mass in 100 parts by mass of the polymerizable monomer (A), and the content of the filler (C) is 50 to 90 parts by mass in total 100 parts by mass of the composition. The method may be a method of using a composition for dental attachments for tooth surface for securing an orthodontic aligner.

### EXAMPLES

The following describes the present invention in greater detail using Examples and Comparative Examples. However, the present invention is not limited to the following descriptions. In the following, "part(s)" means "part(s) by mass", unless otherwise specifically stated.

The components of the compositions for dental attachments of Examples and Comparative Examples are presented below, along with the abbreviations.
Polymerizable monomer (A-1) having an acidic Group
   MDP: 10-Methacryloyloxydecyl dihydrogenphosphate
Polymerizable monomer (A-2) having no acidic group
   Bis-GMA: 2,2-Bis[4-(3-methacryloyloxy-2-hydroxypropoxy)phenyl]propane
   D-2.6E: 2,2-Bis(4-methacryloyloxypolyethoxyphenyl)propane (average number of moles of ethoxy group added: 2.6)
   3G: Triethylene glycol dimethacrylate
   DD: 1,10-Decanediol dimethacrylate
   MAEA: N-Methacryloyloxyethylacrylamide
   DEAA: N,N-Diethylacrylamide
   HEMA: 2-Hydroxyethyl methacrylate
Photopolymerization initiator (B)
   Water-soluble photopolymerization initiator (B-1)
      Li-TPO: Lithium phenyl(2,4,6-trimethylbenzoyl)phosphinate
   Water-insoluble photopolymerization initiator (B-2)
      CQ: dl-Camphorquinone
      BAPO: Bis(2,4,6-trimethylbenzoyl)phenylphosphine oxide
Filler (C)
   Filler 1: Ultrafine particulate silica Aerosil^{®} R 972, manufactured by Nippon Aerosil Co., Ltd.; average particle diameter: 16 nm
   Filler 2: Silane-treated silica
      A three-neck flask was charged with 100 g of OX 50 (ultrafine particulate silica Aerosil^{®} OX 50, manufactured by Nippon Aerosil Co., Ltd.; average particle diameter: 0.04 µm), 7 g of γ-methacryloyloxypropyltrimethoxysilane, and 200 mL of a 0.3 mass% aqueous solution of acetic acid, and the mixture was stirred at room temperature for 2 hours. After removing water by freeze drying, the mixture was subjected to a heat treatment at 80°C for 5 hours to obtain a filler 2.
   Filler 3: Silane-treated silica stone powder
      A silica stone powder (Hi-Silica, manufactured by Nitchitsu Co., Ltd. under this trade name) was pulverized with a ball mill to obtain a pulverized silica stone powder. The pulverized silica stone powder had an average particle diameter of 2.2 µm as measured with a laser diffraction particle size distribution analyzer (Model SALD-2300, manufactured by Shimadzu Corporation). One-hundred parts by mass of the pulverized silica stone powder was surface treated with 4 parts by mass of γ-methacryloyloxypropyltrimethoxysilane by an ordinary method to obtain a silane-treated silica stone powder.
   Filler 4: Silane-treated barium glass powder
      A barium glass (Product Code E-3000, manufactured by Esstech) was pulverized with a ball mill to obtain a barium glass powder. The barium glass powder had an average particle diameter of 2.4 µm as measured with a laser diffraction particle size distribution analyzer (Model SALD-2300, manufactured by Shimadzu Corporation). One-hundred parts by mass of the barium glass powder was surface treated with 3 parts by mass of γ-methacryloyloxypropyltrimethoxysilane by an ordinary method to obtain a silane-treated barium glass powder.
   Filler 5: Silane-treated barium glass powder
      A three-neck flask was charged with 100 g of GM27884 NF180 Grade (a barium glass manufactured by SCHOTT; average particle diameter: 0.18 µm), 13 g of γ-methacryloyloxypropyltrimethoxysilane, and 200 mL of a 0.3 mass% aqueous solution of acetic acid, and the mixture was stirred at room temperature for 2 hours. After removing water by freeze drying, the mixture was subjected to a heat treatment at 80°C for 5 hours to obtain a filler 5.
   Filler 6: Silane-treated barium glass powder
      A three-neck flask was charged with 100 g of 8235 UF0.7 Grade (a barium glass manufactured by SCHOTT; average particle diameter: 0.7 µm), 6 g of γ-methacryloyloxypropyltrimethoxysilane, and 200 mL of a 0.3 mass% aqueous solution of acetic acid, and the mixture was stirred at room temperature for 2 hours. After removing water by freeze drying, the mixture was subjected to a heat treatment at 80°C for 5 hours to obtain a filler 6.
   Filler 7: Silane-treated spherical silica-titania composite oxide powder
      A three-neck flask was charged with 100 g of spherical silica-titania composite oxide (average particle diameter: 0.3 µm), 10 g of γ-methacryloyloxypropyltrimethoxysilane, and 200 mL of a 0.3 mass% aqueous solution of acetic acid, and the mixture was stirred at room temperature for 2 hours. After removing water by freeze drying, the mixture was subjected to a heat treatment at 80°C for 5 hours to obtain a filler 7.
      Ar380: Ultrafine particulate silica Aerosil 380, manufactured by Nippon Aerosil Co., Ltd.; average particle diameter: 7 nm
Polymerization accelerator (D)
   DABE: Ethyl 4-(N,N-dimethylamino)benzoate
Polymerization inhibitor
   BHT: 3,5-di-t-Butyl-4-hydroxytoluene

### Examples 1 to 20 and Comparative Examples 1 to 5 (Preparation of Composition for Dental Attachments)

The raw materials shown in Tables 1 to 3 were mixed at ordinary temperature (23°C) in the dark, and was kneaded to prepare paste-like compositions for dental attachments. The properties of each composition were determined according to the methods of Test Examples 1 to 4 below. The results are presented in Tables 1 to 3.

### Test Example 1: Depth of Photocure

The depth of photocure was evaluated according to JIS T 6514:2015 (Dental Composite Resins for Restoration), specifically as follows. The composition for dental attachments produced was filled into a stainless-steel die (12 mm in thickness, 4 mm in diameter). A film and a glass slide were placed in this order on each side of the die at the top and bottom surfaces, and pressure was applied from both sides. After removing the glass plate from one side, the composition was cured from this side by applying light for 10 seconds through the contacting film surface, using a dental visible-light irradiator PenCure 2000 (manufactured by J. Morita Corp.). After taking out the cured product from the die and wiping off the uncured portion, the cured product was measured for its length from the irradiated surface to the end, using a micrometer (manufactured by Mitsutoyo Corporation). A half value of the measured length was determined as a depth of photocure (n = 5), and a mean value was calculated.

### Test Example 2: Vickers Hardness

A paste of the composition for dental attachments prepared for each Example and Comparative Example was placed on a glass slide in an appropriate amount. With the paste being pressed between glass slides from top and bottom using a 1 mm gauge (manufactured by Mitsutoyo Corporation), light was applied for 10 seconds only from the top with a dental visible-light irradiator PenCure 2000 (manufactured by J. Morita Corp.) to cure the paste and prepare a disc measuring 10 mm in diameter and 1 mm in thickness. After grinding the smooth disc surface with #1500 abrasive paper under dry conditions, the surface was polished to a mirror finish with a diamond paste. The sample prepared in this fashion was then placed under a 200 g load applied for 10 seconds with a micro hardness testing machine (HM-221, manufactured by Mitsutoyo Corporation) for the measurement of Vickers hardness (Hv) (n = 5). A mean value was calculated.

### Test Example 3: Flexural Properties (Flexural modulus, Flexural Strength)

The flexural modulus and flexural strength were evaluated by conducting a flexure test in compliance with ISO 4049:2009, specifically as follows. A paste (composition for dental attachments) prepared was filled into an SUS die (2 mm vertical × 25 mm horizontal × 2 mm in thickness), and the paste was pressed with glass slides from the top and bottom (2 mm × 25 mm surfaces). The paste was then cured by applying light from both sides through the glass slides. Here, light was applied at 5 points each side, 10 seconds at each point, using a dental visible-light irradiator PenCure 2000 (manufactured by J. Morita Corp.). The flexure test was conducted for the cured product at a span length of 20 mm and a crosshead speed of 1 mm/min using a universal testing machine (Autograph AG-I 100kN, manufactured by Shimadzu Corporation) to measure three-point flexural strength and flexural modulus (n = 5). A mean value was calculated.

### Test Example 4: Shear Bond Strength to Tooth Structure (Uncut Enamel of Human Tooth)

The labial surface of an extracted human tooth was brushed with a toothbrush under running water to obtain a sample with a washed tooth structure surface. The sample tooth was secured to a tape attached to the bottom of a mold having 15 holes (a 15-hole mold, manufactured by Ultradent Products Inc.; 35 mm in diameter × 25 mm in height). After filling a plaster into the mold, the plaster was left to stand for about 30 minutes to harden. The sample was taken out of the mold, and brushed with a toothbrush under running water to remove the excess plaster and provide a bonding surface (∅ = 2.38 mm or more). The bonding surface was ultrasonically washed with water for 5 minutes.

A tooth-surface treatment agent 1 (a mixture prepared by mixing 50 parts by mass of concentrated phosphoric acid, 50 parts by mass of distilled water, and 5 parts by mass of Ar 380) was applied to the bonding surface of the sample with a brush. After allowing the agent to stand for 10 seconds, the surface was washed with tap water for 10 seconds, and dried by blowing air.

Subsequently, a separately prepared CR filling mold (Bonding Mold Insert, manufactured by Ultradent Products Inc.; ∅ = 2.38 mm) was installed on a dedicated instrument (Bonding Clamp, manufactured by Ultradent Products Inc.). The sample was secured by lowering the CR filling mold in such a manner that the CR filling mold installed on the dedicated instrument contact the bonding surface of sample treated with the tooth-surface treatment agent 1. Thereafter, the composition for dental attachments of each Example and Comparative Example was filled into the hole of the CR filling mold to form a thin layer of no greater than 1 mm. After filling another portion of the composition for dental attachments into the mold (to about 2/3 of the mold, or about 2 mm thick), the composition was irradiated with light for 10 seconds using a dental visible-light irradiator VALO (manufactured by Ultradent Japan). The sample was removed from the mold, and was used as an adhesion test sample. A total of 20 samples were prepared. The adhesion test samples were immersed in distilled water, and were left to stand for 24 hours in this state in a thermostatic chamber with the chamber temperature set to 37°C. The samples were taken out of the chamber, and, for evaluation of initial bond strength, ten of the twenty samples were immediately measured for bond strength after being kept in the chamber for 24 hours. The mean values of the measurement results are shown in Tables 1 to 3 as "Initial Bond Strength". For evaluation of bond durability, the remaining ten samples were measured for bond strength after 10,000 cycles of a thermal process by alternately immersing the samples for 1 minute in 4°C cold water and in 60°C hot water. The mean values of the measurement results are shown in Tables 1 to 3 as "Bond Durability". For the measurement of shear bond strength, the adhesion test sample was installed on a dedicated holder (Test Base Clamp, manufactured by Ultradent Products Inc.), and the shear bond strength was measured with a dedicated jig (Crosshead Assembly, manufactured by Ultradent Products Inc.) and a universal testing machine (Autograph AG-I 100kN, manufactured by Shimadzu Corporation) with the crosshead speed set at 1 mm/min. The mean value of measured values for ten adhesion test samples was determined as the bond strength. This testing method is preferable because it enables an evaluation using a specimen that is about 2 mm thick, close to the thickness of an actual dental attachment, and that is similar in form to a dental attachment.

### Test Example 5: Tensile Bond Strength to Prosthesis (Zirconia or Gold Silver Palladium Alloy)

A cylindrical zirconia sintered body (12 mm in inner diameter × 5 mm in height; fired at 1,500°C for 2 hours) was prepared as an adherend from a zirconia disc for CAD/CAM system (KATANA^{®} Zirconia HT, manufactured by Kuraray Noritake Dental Inc. under this trade name). A gold silver palladium alloy cast body (Castwell M.C. <Gold 12%>, manufactured by GC) of a shape with 10 mm × 10 mm × 1 mm dimensions was also used as an adherend. The surface of each adherend was ground with #1000 silicon carbide paper (manufactured by Nihon Kenshi Co., Ltd.) to provide a flat surface, and the surface was dried by removing water by air blowing. After drying, an about 150 µm-thick adhesive tape having a 5 mm circular hole was attached to the dried flat surface to define a bonding area. The composition for dental attachments of each Example and Comparative Example was then applied by filling the circular hole, and the hole was covered with a release film (polyester). A glass slide was placed on the release film, and pressure was applied to flatten the surface of the applied composition for dental attachments. The composition for dental attachments was then cured into a cured product by applying light for 10 seconds through the release film using a dental visible-light irradiator VALO (manufactured by Ultradent Japan). A cylindrical stainless steel rod (7 mm in diameter, 2.5 cm in length) was then bonded at one end (circular cross section) to the surface of the cured product, using a commercially available dental resin cement (Panavia^{®} 21, manufactured by Kuraray Noritake Dental Inc. under this trade name). After bonding, the sample was left to stand at room temperature for 30 minutes, and was immersed in distilled water to obtain an adhesion test sample. Twenty samples were prepared for the zirconia sintered body and for the gold silver palladium alloy cast body. The samples were immersed in distilled water, and left to stand for 24 hours in a thermostatic chamber held at 37°C. For evaluation of initial bond strength, ten of the twenty samples were immediately measured for bond strength after being kept in the chamber for 24 hours. The mean values of the measurement results are shown in Tables 1 to 3 as "Initial Bond Strength". The tensile bond strength indicates the initial bond strength after bonding. For evaluation of bond durability, the remaining ten samples were measured for bond strength after 10,000 cycles of a thermal process by alternately immersing the samples for 1 minute in 4°C cold water and in 60°C hot water. The mean values of the measurement results are shown in Tables 1 to 3 as "Bond Durability". The tensile bond strength was measured with a universal testing machine (manufactured by Shimadzu Corporation) with the crosshead speed set at 2 mm/min. The mean value of measured values for ten adhesion test samples was determined as the bond strength.

**[Table 1]**

| Components (parts by mass) | | | Ex. 1 | Ex. 2 | Ex. 3 | Ex. 4 | Ex. 5 | Ex. 6 | Ex. 7 | Ex. 8 | Ex. 9 | Ex. 10 |
|---|---|---|---|---|---|---|---|---|---|---|---|---|
| Polymerizable monomer (A-1) having acidic group | | MDP | 10 | 10 | 10 | 10 | 10 | 10 | 10 | 10 | 10 | 10 |
| Polymerizable monomer (A-2) having no acidic group | (A-2b) | Bis-GMA | 30 | 30 | 30 | 30 | 30 | 30 | 30 | 30 | 30 | - |
| | | D-2.6E | - | - | - | - | - | - | - | - | - | 30 |
| | | 3G | 50 | 50 | 50 | 50 | 50 | 50 | 50 | 50 | 50 | 50 |
| | | DD | - | - | - | - | - | - | - | - | - | - |
| | (A-2a) | MAEA | 10 | 10 | 10 | 10 | 10 | 10 | 10 | 10 | 10 | 10 |
| | (A-2c) | HEMA | - | - | - | - | - | - | - | - | - | - |
| | | DEAA | - | - | - | - | - | - | - | - | - | - |
| Photopolymerization initiator (B) | (B-1) | Li-TPO | - | - | - | - | - | - | - | - | - | - |
| | (B-2) | CQ | 0.2 | 0.2 | 0.2 | 0.2 | 0.2 | 0.2 | 0.2 | 0.2 | 0.2 | 0.2 |
| | | BAPO | - | - | - | - | - | - | - | - | - | - |
| Filler (C) | | Filler 1 | 10 | 10 | 10 | 10 | 10 | - | - | 25 | 40 | 10 |
| | | Filler 2 | - | - | - | - | - | - | 10 | - | - | - |
| | | Filler 3 | 250 | - | - | - | - | - | 250 | 250 | 250 | 250 |
| | | Filler 4 | - | 250 | - | - | - | - | - | - | - | - |
| | | Filler 5 | - | - | 180 | - | - | 90 | - | - | - | - |
| | | Filler 6 | - | - | - | 200 | - | 100 | - | - | - | - |
| | | Filler 7 | - | - | - | - | 180 | - | - | - | - | - |
| Polymerization accelerator (D) | | DABE | 0.4 | 0.4 | 0.4 | 0.4 | 0.4 | 0.4 | 0.4 | 0.4 | 0.4 | 0.4 |
| Other | | BHT | 0.05 | 0.05 | 0.05 | 0.05 | 0.05 | 0.05 | 0.05 | 0.05 | 0.05 | 0.05 |
| Depth of photocure | | mm | 3.1 | 3.2 | 3.2 | 3.8 | 3.8 | 3.4 | 2.7 | 2.4 | 2.2 | 3.1 |
| Vickers hardness | | Hv | 40 | 32 | 43 | 33 | 45 | 38 | 42 | 41 | 44 | 39 |
| Flexural strength | | MPa | 132 | 125 | 119 | 121 | 130 | 123 | 125 | 123 | 130 | 123 |
| Flexural modulus | | GPa | 8.8 | 8.0 | 6.8 | 7.3 | 7.8 | 7.8 | 8.5 | 8.3 | 9.0 | 7.5 |
| Shear bond strength to uncut enamel of human tooth | | | | | | | | | | | | |
| Initial bond strength | | MPa | 25 | 24 | 24 | 23 | 24 | 20 | 24 | 21 | 19 | 26 |
| Bond durability | | MPa | 27 | 26 | 25 | 26 | 25 | 21 | 25 | 23 | 22 | 28 |
| Tensile bond strength to zirconia | | | | | | | | | | | | |
| Initial bond strength | | MPa | 25 | 24 | 26 | 21 | 23 | 19 | 23 | 20 | 18 | 27 |
| Bond durability | | MPa | 19 | 19 | 20 | 15 | 18 | 14 | 17 | 14 | 13 | 22 |
| Tensile bond strength to gold silver palladium alloy | | | | | | | | | | | | |
| Initial bond strength | | MPa | 18 | 17 | 18 | 14 | 15 | 13 | 16 | 14 | 13 | 19 |
| Bond durability | | MPa | 12 | 11 | 13 | 10 | 11 | 9 | 12 | 9 | 9 | 15 |

**[Table 2]**

| Components (parts by mass) | | | Ex. 11 | Ex. 12 | Ex. 13 | Ex. 14 | Ex. 15 | Ex. 16 | Ex. 17 | Ex. 18 | Ex. 19 | Ex. 20 |
|---|---|---|---|---|---|---|---|---|---|---|---|---|
| Polymerizable monomer (A-1) having acidic group | | MDP | 10 | 10 | 30 | 10 | 10 | 10 | 10 | 10 | 10 | 10 |
| Polymerizable monomer (A-2) having no acidic group | (A-2b) | Bis-GMA | 30 | 50 | 30 | - | - | - | 30 | 30 | 40 | 30 |
| | | D-2.6E | - | - | - | 30 | 30 | 30 | - | - | - | - |
| | | 3G | - | 30 | 30 | 40 | 40 | 15 | - | - | 50 | 50 |
| | | DD | 50 | - | - | - | - | - | 50 | 50 | - | 10 |
| | (A-2a) | MAEA | 10 | 10 | 10 | 10 | 10 | 10 | 10 | 10 | - | - |
| | (A-2c) | HEMA | - | - | - | 10 | - | 25 | - | - | - | - |
| | | DEAA | - | - | - | - | 10 | - | - | - | - | - |
| Photopolymerization initiator (B) | (B-1) | Li-TPO | - | - | - | - | - | - | 0.3 | - | - | - |
| | (B-2) | CQ | 0.2 | 0.2 | 0.2 | 0.2 | 0.2 | 0.2 | 0.2 | 0.2 | 0.2 | 0.2 |
| | | BAPO | - | - | - | - | - | - | - | 0.2 | - | - |
| Filler (C) | | Filler 1 | 10 | 10 | 10 | 10 | 10 | 10 | 10 | 10 | 10 | 10 |
| | | Filler 2 | - | - | - | - | - | - | - | - | - | - |
| | | Filler 3 | 250 | 250 | 250 | 250 | 250 | 250 | 250 | 250 | 250 | 250 |
| | | Filler 4 | - | - | - | - | - | - | - | - | - | - |
| | | Filler 5 | - | - | - | - | - | - | - | - | - | - |
| | | Filler 6 | - | - | - | - | - | - | - | - | - | - |
| | | Filler 7 | - | - | - | - | - | - | - | - | - | - |
| Polymerization accelerator (D) | | DABE | 0.4 | 0.4 | 0.4 | 0.4 | 0.4 | 0.4 | 0.4 | 0.4 | 0.4 | 0.4 |
| Other | | BHT | 0.05 | 0.05 | 0.05 | 0.05 | 0.05 | 0.05 | 0.05 | 0.05 | 0.05 | 0.05 |
| Depth of photocure | | mm | 2.9 | 3.6 | 2.8 | 2.8 | 2.8 | 2.5 | 3.2 | 3.2 | 3.6 | 3.2 |
| Vickers hardness | | Hv | 37 | 38 | 34 | 30 | 32 | 26 | 42 | 43 | 45 | 43 |
| Flexural strength | | MPa | 120 | 136 | 110 | 102 | 104 | 88 | 133 | 138 | 140 | 132 |
| Flexural modulus | | GPa | 5.5 | 8.7 | 6.5 | 7.5 | 7.8 | 6.4 | 9.1 | 9.6 | 9.4 | 8.3 |
| Shear bond strength to uncut enamel of human tooth | | | | | | | | | | | | |
| Initial bond strength | | MPa | 23 | 21 | 18 | 24 | 25 | 22 | 29 | 26 | 22 | 24 |
| Bond durability | | MPa | 25 | 23 | 20 | 26 | 25 | 24 | 32 | 27 | 23 | 24 |
| Tensile bond strength to zirconia | | | | | | | | | | | | |
| Initial bond strength | | MPa | 22 | 20 | 17 | 23 | 26 | 24 | 27 | 25 | 20 | 24 |
| Bond durability | | MPa | 17 | 15 | 12 | 18 | 21 | 20 | 23 | 20 | 15 | 20 |
| Tensile bond strength to gold silver palladium alloy | | | | | | | | | | | | |
| Initial bond strength | | MPa | 16 | 15 | 14 | 17 | 19 | 17 | 18 | 16 | 15 | 15 |
| Bond durability | | MPa | 10 | 11 | 10 | 12 | 13 | 13 | 12 | 11 | 9 | 10 |

**[Table 3]**

| Components (parts by mass) | | | Com. Ex. 1 | Com. Ex. 2 | Com. Ex. 3 | Com. Ex. 4 | Com. Ex. 5 |
|---|---|---|---|---|---|---|---|
| Polymerizable monomer (A-1) having acidic group | | MDP | 10 | 10 | 10 | 0 | 50 |
| Polymerizable monomer (A-2) having no acidic group | (A-2b) | Bis-GMA | 30 | 30 | 30 | 30 | 20 |
| | | D-2.6E | | | | | |
| | | 3G | 50 | 50 | 50 | 60 | 30 |
| | | DD | - | - | - | - | - |
| | (A-2a) | MAEA | 10 | 10 | 10 | 10 | 10 |
| Photopolymerization initiator (B) | (B-1) | Li-TPO | - | - | - | - | - |
| | (B-2) | CQ | 0.2 | 0.2 | 0.2 | 0.2 | 0.2 |
| | | BAPO | - | - | - | - | - |
| Filler (C) | | Filler 1 | 15 | 15 | 10 | 10 | 10 |
| | | Filler 2 | - | - | - | - | - |
| | | Filler 3 | 30 | 60 | 990 | 240 | 240 |
| | | Filler 4 | - | - | - | - | - |
| | | Filler 5 | - | - | - | - | - |
| | | Filler 6 | - | - | - | - | - |
| | | Filler 7 | - | - | - | - | - |
| Polymerization accelerator (D) | | DABE | 0.4 | 0.4 | 0.4 | 0.4 | 0.4 |
| Other | | BHT | 0.05 | 0.05 | 0.05 | 0.05 | 0.05 |
| Depth of photocure | | mm | 4.0 | 3.8 | 2.6 | 3.5 | 2.2 |
| Vickers hardness | | Hv | 28 | 30 | 50 | 44 | 30 |
| Flexural strength | | MPa | 103 | 112 | 165 | 160 | 90 |
| Flexural modulus | | GPa | 2.2 | 2.8 | 13.1 | 11.1 | 4.5 |
| Shear bond strength to uncut enamel of human tooth | | | | | | | |
| Initial bond strength | | MPa | 27 | 26 | 12 | 1 | 12 |
| Bond durability | | MPa | 30 | 29 | 11 | 0 | 11 |
| Tensile bond strength to zirconia | | | | | | | |
| Initial bond strength | | MPa | 27 | 25 | 18 | 10 | 12 |
| Bond durability | | MPa | 22 | 21 | 12 | 0 | 5 |
| Tensile bond strength to gold silver palladium alloy | | | | | | | |
| Initial bond strength | | MPa | 18 | 17 | 14 | 9 | 8 |
| Bond durability | | MPa | 12 | 11 | 7 | 0 | 0 |

The compositions for dental attachments of Examples had a depth of photocure of 2.2 mm or more, and the cured products of these compositions had a Vickers hardness of 32 Hv or more, and a flexural modulus of 5.5 GPa to 9.6 GPa. It can be seen from these results presented in Tables 1 and 2 that the compositions for dental attachments of Examples excel in mechanical strength, and have an excellent initial bond strength (shear bond strength) of 18 MPa or more for uncut enamel, and an excellent bond durability of 20 MPa. It was confirmed that the compositions of Examples exhibit excellent initial bond strength to uncut enamel while having excellent bond durability, despite that the outermost surface of enamel is more acid resistant than the enamel in the tooth structure, and cannot be demineralized as easily by etching, and the surface of uncut enamel tends to have a lower bond strength than cut enamel surfaces. It can also be seen that the compositions of Examples have an excellent bond strength (tensile bond strength) of 13 MPa or more for zirconia and gold silver palladium alloy, and an excellent bond durability of 9 MPa or more. This is in contrast to the compositions for dental attachments of Comparative Examples. The flexural modulus was less than 3.0 GPa, and the mechanical strength was insufficient in Comparative Examples 1 and 2, in which the content of filler (C) was outside the range of the present invention, as shown in Table 3. In Comparative Example 3 in which the content of filler (C) was also outside the range of the present invention, the composition was shown to have a low shear bond strength of 12 MPa or less for uncut enamel, and a low bond durability of 11 MPa or less. In Comparative Examples 4 and 5 in which the polymerizable monomer having an acidic group did not have the mass ratio specified by the present invention, the compositions were shown to have a low shear bond strength of 12 MPa or less for uncut enamel, and a low bond durability of 11 MPa or less. The compositions of Comparative Examples 4 and 5 were also shown to have a low initial bond strength (tensile bond strength) of 12 MPa or less for zirconia and gold silver palladium alloy, and a low bond durability of 5 MPa or less.

### INDUSTRIAL APPLICABILITY

A composition for dental attachments of the present invention can be suitably used as a dental attachment for aligner orthodontics.

## Claims

1. A composition for dental attachments, comprising a polymerizable monomer (A), a photopolymerization initiator (B), and a filler (C),
the polymerizable monomer (A) comprising a polymerizable monomer (A-1) having an acidic group, and a polymerizable monomer (A-2) having no acidic group,
the content of the polymerizable monomer (A-1) having an acidic group being 1 to 40 parts by mass in total 100 parts by mass of the polymerizable monomer (A),
the content of the filler (C) being 50 to 90 parts by mass in total 100 parts by mass of the composition.

2. The composition for dental attachments according to claim 1, wherein the polymerizable monomer (A-2) having no acidic group comprises a hydrophobic polymerizable monomer (A-2b) having no acidic group, and, optionally, a hydrophilic polymerizable monomer (A-2c) having no acidic group,
the hydrophilic polymerizable monomer (A-2c) having no acidic group and the hydrophobic polymerizable monomer (A-2b) having no acidic group having a mass ratio of 0:10 to 2:1 as a mass ratio of the hydrophilic polymerizable monomer (A-2c) having no acidic group to the hydrophobic polymerizable monomer (A-2b) having no acidic group.

3. The composition for dental attachments according to claim 1 or 2, wherein the composition for dental attachments is of a one-pack type.

4. The composition for dental attachments according to any one of claims 1 to 3, wherein the polymerizable monomer (A-1) having an acidic group is a polymerizable monomer having a phosphoric acid group, and/or a polymerizable monomer having a carboxylic acid group.

5. The composition for dental attachments according to any one of claims 1 to 4, wherein the polymerizable monomer (A-1) having an acidic group is 10-methacryloyloxydecyl dihydrogenphosphate.

6. The composition for dental attachments according to any one of claims 2 to 5, wherein the hydrophilic polymerizable monomer (A-2c) having no acidic group and the hydrophobic polymerizable monomer (A-2b) having no acidic group have a mass ratio of 0:10 to 1:1 as a mass ratio of the hydrophilic polymerizable monomer (A-2c) having no acidic group to the hydrophobic polymerizable monomer (A-2b) having no acidic group.

7. The composition for dental attachments according to any one of claims 2 to 5, wherein the hydrophilic polymerizable monomer (A-2c) having no acidic group and the hydrophobic polymerizable monomer (A-2b) having no acidic group have a mass ratio of 0:10 to 1:2 as a mass ratio of the hydrophilic polymerizable monomer (A-2c) having no acidic group to the hydrophobic polymerizable monomer (A-2b) having no acidic group.

8. The composition for dental attachments according to any one of claims 1 to 7, wherein the filler (C) comprises at least one combination selected from the group consisting of:
a combination (I) of a filler (C-1) having an average particle diameter of 1 nm or more and less than 0.1 µm, and a filler (C-2) having an average particle diameter of 0.1 µm or more and 1 µm or less;
a combination (II) of a filler (C-1) having an average particle diameter of 1 nm or more and less than 0.1 µm, and a filler (C-3) having an average particle diameter of more than 1 µm and 10 µm or less;
a combination (III) of a filler (C-1) having an average particle diameter of 1 nm or more and less than 0.1 µm, a filler (C-2) having an average particle diameter of 0.1 µm or more and 1 µm or less, and a filler (C-3) having an average particle diameter of more than 1 µm and 10 µm or less; and
a combination (IV) of fillers (C-2) having an average particle diameter of 0.1 µm or more and 1 µm or less.

9. The composition for dental attachments according to claim 8, wherein the filler (C) comprises the combination (I) or the combination (II).

10. The composition for dental attachments according to any one of claims 1 to 9, wherein a cured product of the composition has a flexural modulus of 3 GPa or more.

11. The composition for dental attachments according to any one of claims 1 to 10, wherein the photopolymerization initiator (B) comprises a water-soluble photopolymerization initiator (B-1).

12. The composition for dental attachments according to any one of claims 1 to 11, wherein the photopolymerization initiator (B) comprises a water-insoluble photopolymerization initiator (B-2).

13. The composition for dental attachments according to any one of claims 1 to 12, wherein the polymerizable monomer (A-2) having no acidic group comprises an asymmetric acrylamide-methacrylic acid ester compound (A-2a) represented by the following general formula (1), 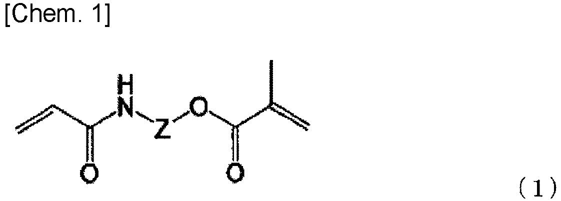 wherein Z is an optionally substituted C₁ to Ca linear or branched aliphatic group, or an optionally substituted aromatic group, and the aliphatic group may be interrupted by at least one binding group selected from the group consisting of -O-, -S-, -CO-, -CO-O-, -O-CO-, -NR¹-, -CO-NR'-, -NR'-CO-, -CO-O-NR'-, -O-CO-NR¹-, and -NR'-CO-NR'-, where R¹ represents a hydrogen atom, or an optionally substituted C₁ to Ca linear or branched aliphatic group.

14. The composition for dental attachments according to claim 13, wherein Z is an optionally substituted C₁ to C₄ linear or branched aliphatic group.

15. The composition for dental attachments according to claim 13 or 14, wherein Z is an optionally substituted C₁ to C₄ linear or branched alkylene group.

16. The composition for dental attachments according to any one of claims 13 to 15, wherein the asymmetric acrylamide-methacrylic acid ester compound (A-2a) represented by the general formula (1) is at least one selected from the group consisting of N-methacryloyloxyethylacrylamide, N-methacryloyloxypropylacrylamide, N-methacryloyloxybutylacrylamide, N-(1-ethyl-(2-methacryloyloxy)ethyl)acrylamide, and N-(2-(2-methacryloyloxyethoxy)ethyl)acrylamide.
